# EUROPEAN PATENT SPECIFICATION

(11) **EP 1 049 676 B1**
(45) Date of publication and mention of the grant of the patent: **12.10.2005**
(21) Application number: 98964871.2
(22) Date of filing: 23.12.1998
(51) Int. Cl.: C07D 211/58, A61K 31/445, C07D 405/12, C07D 405/06, C07D 409/06, C07D 401/06, A61K 31/535

(54) **4- ARYL(PIPERIDIN-4-YL)] AMINOBENZAMIDES WHICH BIND TO THE DELTA-OPIOID RECEPTOR**
(4)-[ARYL(PIPERIDIN-4-YL) AMINOBENZAMIDDERIVATE DIE AN DELTA-OPIOID REZEPTOR BINDEN
(4)-ARYL(PIPERIDINE-4-YL) AMINOBENZAMIDES SE LIANT AU RECEPTEUR OPIOIDE DELTA

(30) Priority: 24.12.1997 US 68794 P
(43) Date of publication of application: 08.11.2000
(73) Proprietor: Ortho-McNeil Pharmaceutical, Inc., Raritan, NJ 08869-0602 (US)
(72) Inventor: CARSON, John, R., Norristown, PA 19403 (US); CARMOSIN, Richard, J., Quakertown, PA 18851 (US); FITZPATRICK, Louis, J., Souderton, PA 18964 (US); REITZ, Allen, B., Lansdale, PA 19446 (US); JETTER, Michele, C., Norristown, PA 19403 (US)
(74) Representative: Mercer, Christopher Paul
(86) International application number: PCT/US1998/027350
(87) International publication number: WO 1999/033806

(56) References cited:
- EP-A- 0 383 579
- WO-A-93/15062
- WO-A-96/36620
- WO-A-97/10230
- WO-A-97/23466
- WO-A-98/28270

## Description

The present invention relates to delta-opioid receptor agonists/antagonists. More particularly, the present invention relates to 4-[aryl(piperidin-4-yl)]aminobenzamides which are delta-opioid receptor agonists useful as analgesics.

### BACKGROUND OF THE INVENTION

WO9723466 to Plobeck N. et al., discloses compounds (approximately) of the formula: 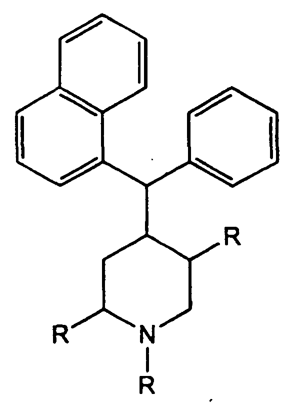 which are mu-opioid antagonists.

WO9636620 to Dondio G., discloses compounds (most relevantly) of the formula: 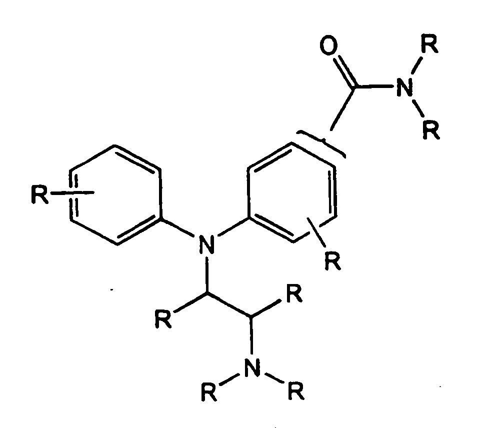 which are delta-opioid agonists/antagonists.

WO9710230 to Dondio G. et al., discloses compounds (most relevantly) of the formula: 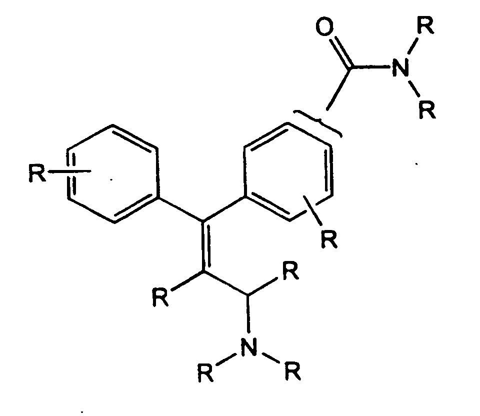 which are delta-opioid, kappa-opioid and mu-opioid receptor agonists/antagonists.

WO9315062 to Chang K. et al., discloses compounds (approximately) of the formula: 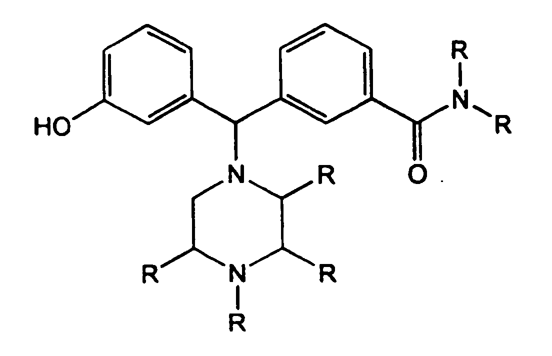 which are delta-opioid and mu-opioid receptor agonists.

It is an object of the present invention to provide delta-opioid receptor agonists as analgesics.

It is another object of the present invention to provide delta-opioid receptor selective agonists as analgesics having reduced side-effects.

It is another object of the present invention to provide delta-opioid receptor agonists/antagonists as immunosuppressants, antiinflammatory agents, agents for the treatment of mental illness, medicaments for drug and alcohol abuse, agents for treating gastritis and diarrhea, cardiovascular agents, and agents for the treatment of respiratory diseases.

It is another object of the present invention to provide delta-opioid receptor selective agonists/antagonists as immunosuppressants, antiinflammatory agents, agents for the treatment of mental illness, medicaments for drug and alcohol abuse, agents for treating gastritis and diarrhea, cardiovascular agents, and agents for the treatment of respiratory diseases, having reduced side-effects.

### SUMMARY OF THE INVENTION

There are provided by the present invention delta-opioid receptor agonists/antagonists of the general formula: 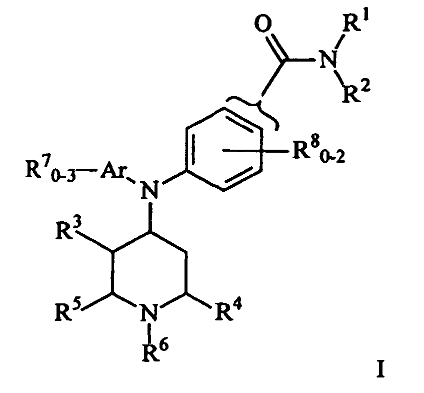 where
- Ar: is phenyl, 1-naphthyl or 2-naphthyl, each optionally substituted with 1 to 3 R⁷;
- R¹ and R²: are independently selected from the group consisting of hydrogen, C_{1 8}alkyl; phenyl, optionally mono-, di-, or tri-substituted with halo, C₁₋₆alkyl, C₁₋₆alkoxy or trifluoromethyl; or benzyl, optionally mono-, di-, or tri-substituted with halo, C₁₋₆alkyl, C₁₋₆alkoxy or trifluoromethyl, or alternatively, R¹ and R² are taken together with their N of attachment to form a ring which is selected from the group consisting of pyrrolidinyl, morpholinyl, piperidinyl and hexamethyleneiminyl, each said ring optionally substituted with 1 to 4 methyl groups;
- R³: is selected from hydrogen and C₁₋₄alkyl;
- R⁴ and R⁵: are hydrogen;
- R⁶: is selected from the group consisting of hydrogen; C₁₋₈alkyl; C₃₋₆cycloalkylC₁₋₃alkyl; C₃₋₆alkenyl; C₁₋₆alkoxyC₁₋₃alkyl; and phenylC₁₋₄alkyl, where the phenyl is optionally mono-, di-, or tri-substituted with R⁷;
- R⁷: is independently selected from the group consisting of hydroxy, halo, C₁₋₃alkyl, C₁₋₃alkoxy, C₁₋₃acyl, C₁₋₃acyloxy, cyano, amino, C₁₋₃acylamino, C₁₋₃alkylamino, di(C₁₋₃alkyl)amino, C₁₋₃alkylthio, C₁₋₃alkylsulfonyl, trifluoromethyl and trifluoromethoxy, and two R⁷ can together form a single moiety selected from the group consisting of -(CH₂)₃₋₅- and -O(CH₂)₁₋₃O- attached to adjacent carbon atoms of Ar; and
- R⁸: is independently selected from the group consisting of halo, C₁₋₆alkyl, C₁₋₆alkoxy and trifluoromethyl;
wherein the compounds of the formulae: 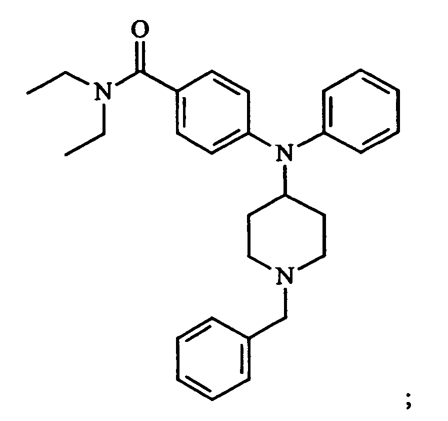 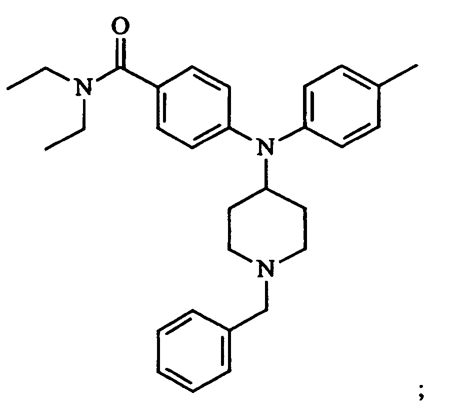 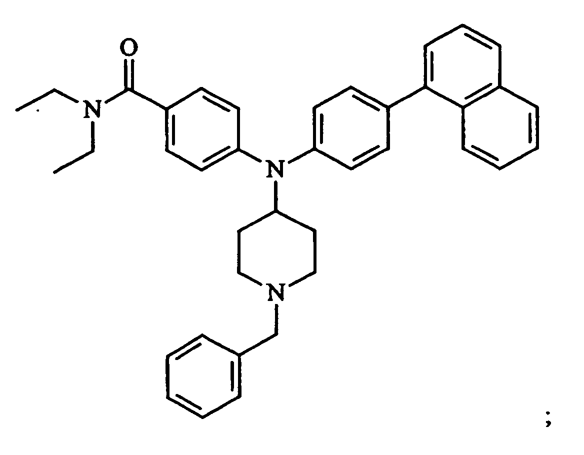 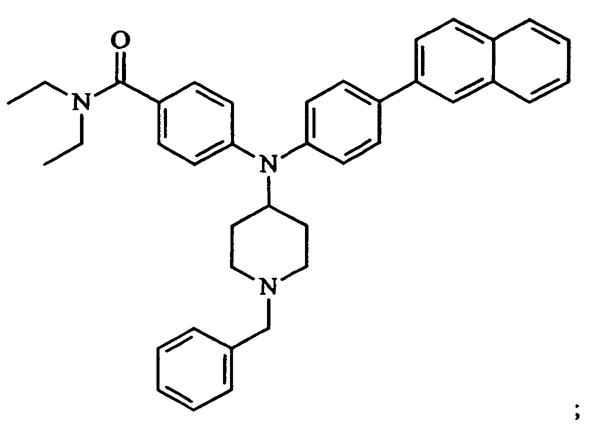 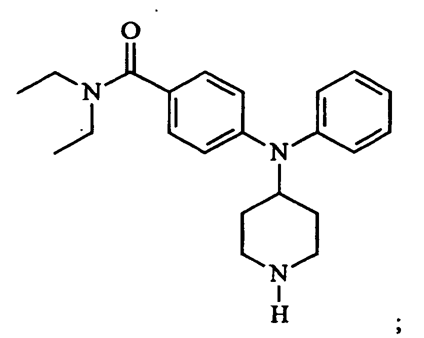 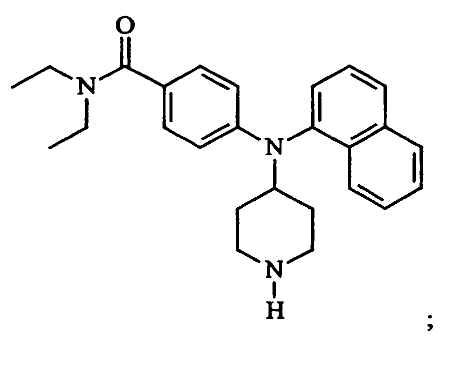 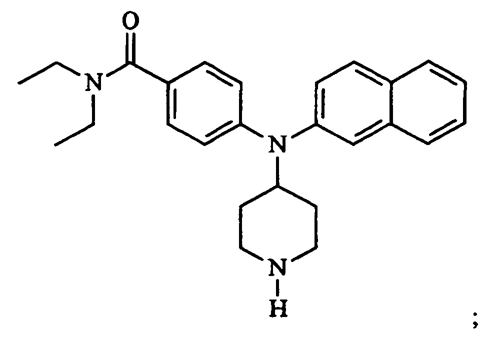 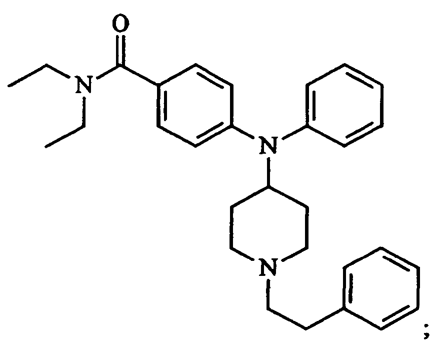 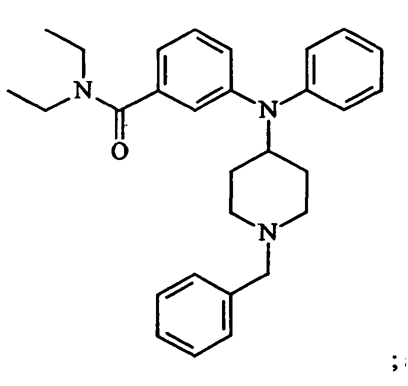 and 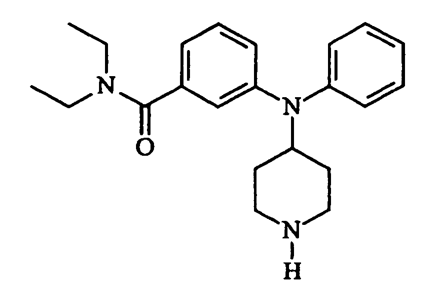 are disclaimed.

As delta-opioid receptor agonists. such compounds are useful as analgesics. Depending on their agonist/antagonist effect. such compounds may also be useful immunosuppressants, antiiinflammantory agents, agents for treatment of mental illness, medicaments for drug and alcohol abuse, agents for treating gastritis and diarrhea, cardiovascular agents, and agents for the treatment of respiratory diseases.

### DETAILED DESCRIPTION OF THE INVENTION

The core structure of the compounds of the present invention can be made in a two step process. This process must be modified as required by the strategy employed to obtain the various substituents. In a first strategy, the starting materials are substituted as desired with the final substituents and, where the substituents or their protected forms are stable to the reaction conditions, the core structure may be subsequently made by the two step process. In a second strategy, the final core structure is obtained and, where the core structure is stable to the modifying reaction conditions, the substituents are modified as desired. Variations might include modifying the substituents on intermdiates or replacing precursor substituents on the finished core structure.

Scheme A generally describes the manufacture of the compounds of the present invention. The first step of Scheme A is a reductive alkylation of piperidone A1 and amine A2 to produce N-aryl-piperidineamine A3. The reductive alkylation is catried out by combining the ketone A1, amine A2, and an appropriate solvent/reducing agent combination to form a reaction mixture which is cooled or heated as necessary. Suitable solvent/reducing agent combinations include 1,2-dichloroethane or acetonitrilelNaBH(OAc)₃ + acid catalyst; methanol/NaBH₃CN + acid catalyst; methanol or ethanol or isopropanol/NaBH₄; or alcoholic solvent/H₂ + noble metal catalyst. The use of the 1,2-dichloroethane or acetonitfile/NaBH(OAc)₃ + acid catalyst combination is further described by Abdel-Magid, A. F., et al., J. Org.Chem., Vol. 61, pp 3849-3862 (1996). In the second step of Scheme A, the N-aryl-piperidineamine A3 is reacted with a bromo, iodo or trifluoromethanesulfonyloxy substituted benzamide A4 in the presence of a palladium catalyst, phosphine ligand and base to give the (N-aryl, N-piperidin-4-yl)aminobenzamide. Preferred palladium catalysts include PdCl₂ + phosphine ligand, tris(dibenzylideneacetone)dipalladium(0) which is Pd₂(dba)₃ + phosphine ligand, Pd(OAc)₂ + phosphine ligand and Pd(Ph₃P)₄(0). Suitable phosphine ligands include BTNAP and tri(o-tolyl phosphine). Suitable bases include NaOtBu and Cs₂CO₃. The reaction of the second step is an arylation further described by Buchwald, S. L., J. Org. Chem., Vol. 61, p 1133 (1996). The manufacture of the various starting materials for Scheme A is well within the skill of persons versed in this art.

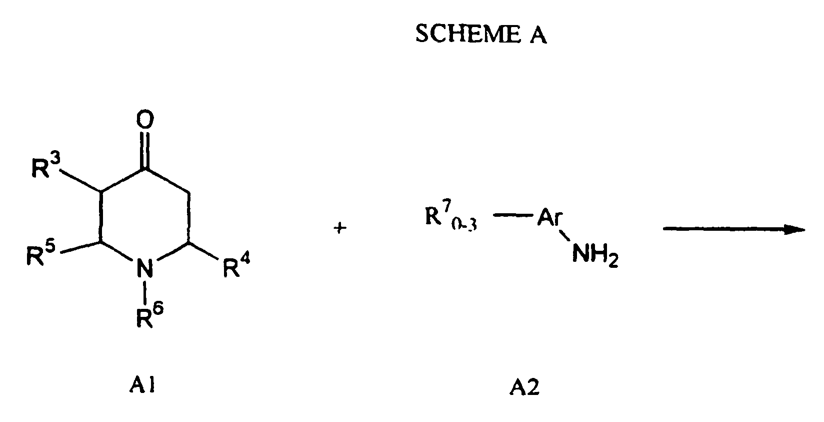

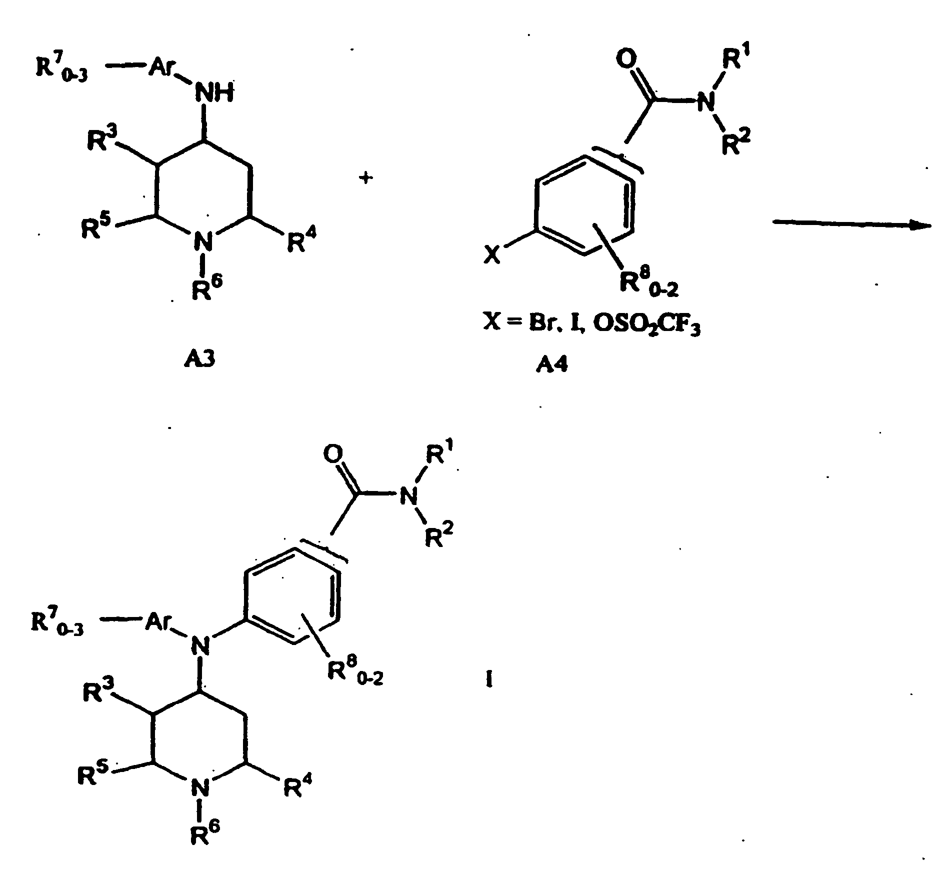

Preferred Ar is phenyl.

Preferred R¹ and R² are independently selected from the group consisting of hydrogen, methyl, ethyl, n-propyl, i-propyl, n-butyl, t-butyl, i-butyl, phenyl, p-chlorophenyl, p-fluorophenyl, p-methylphenyl, p-trifluoromethylphenyl, benzyl, p-chlorobenzyl, p-fluorobenzyl, p-methylbenzyl and p-trifluoromethylbenzyl, or alternatively, preferred R¹ and R² are taken together with their N of attachment to form a ring which is selected from the group consisting of pyrrolidinyl and piperidinyl.

Preferred R³ are selected from hydrogen. methyl, ethyl, n-propyl, i-propyl and t-butyl.

Preferred R⁶ are selected from the group consisting of hydrogen, methyl, ethyl, n-propyl, i-propyl, n-butyl, i-butyl, t-butyl, cyclopropylmethyl, ethenyl, allyl, methoxymethyl, benzyl, p-chlorobenzyl, p-fluorobenzyl, p-methylbenzyl, p-trifluoromethylbenzyl, p-aminobenzyl, and phenylCH₂CH₂-, any of which may be R⁷ substituted as taught above. It is a preferred embodiment of R⁶, that where it contains a phenyl group, that the moiety linking the phenyl group to the piperidinyl moiety be at least two carbon atoms long. Thus this linking moiety might be ethyl or propyl which is beta substituted with the phenyl group, or propyl, which is gamma substituted.

Preferred R⁷ are independently selected from the group consisting of hydroxy, chloro, bromo, fluoro, methyl, ethyl, n-propyl, i-propyl, n-butyl, i-butyl, t-butyl, methoxy, ethoxy, formyl, acyl, acetoxy, cyano, amino, methylamido, methylamino, N,N-dimethylamino, methylthio, methylsulfonyl, trifluoromethoxy and trifluoromethyl, and preferred moieties where two R⁷ together form a single moiety are selected from the group consisting of propylene. butylene and -OCH₂O-.

Preferred R⁸ are independently selected from the group consisting of chloro, bromo, fluoro, methyl, ethyl, n-propyl, i-propyl, t-butyl, methoxy, ethoxy and trifluoromethyl.

Preferred compounds of the present invention have the general structure: 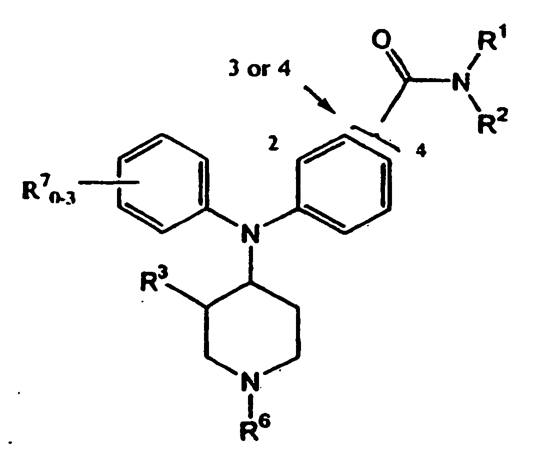 where R¹, R², R³, R⁶ and R⁷ are dependently selected from the groups consisting of:

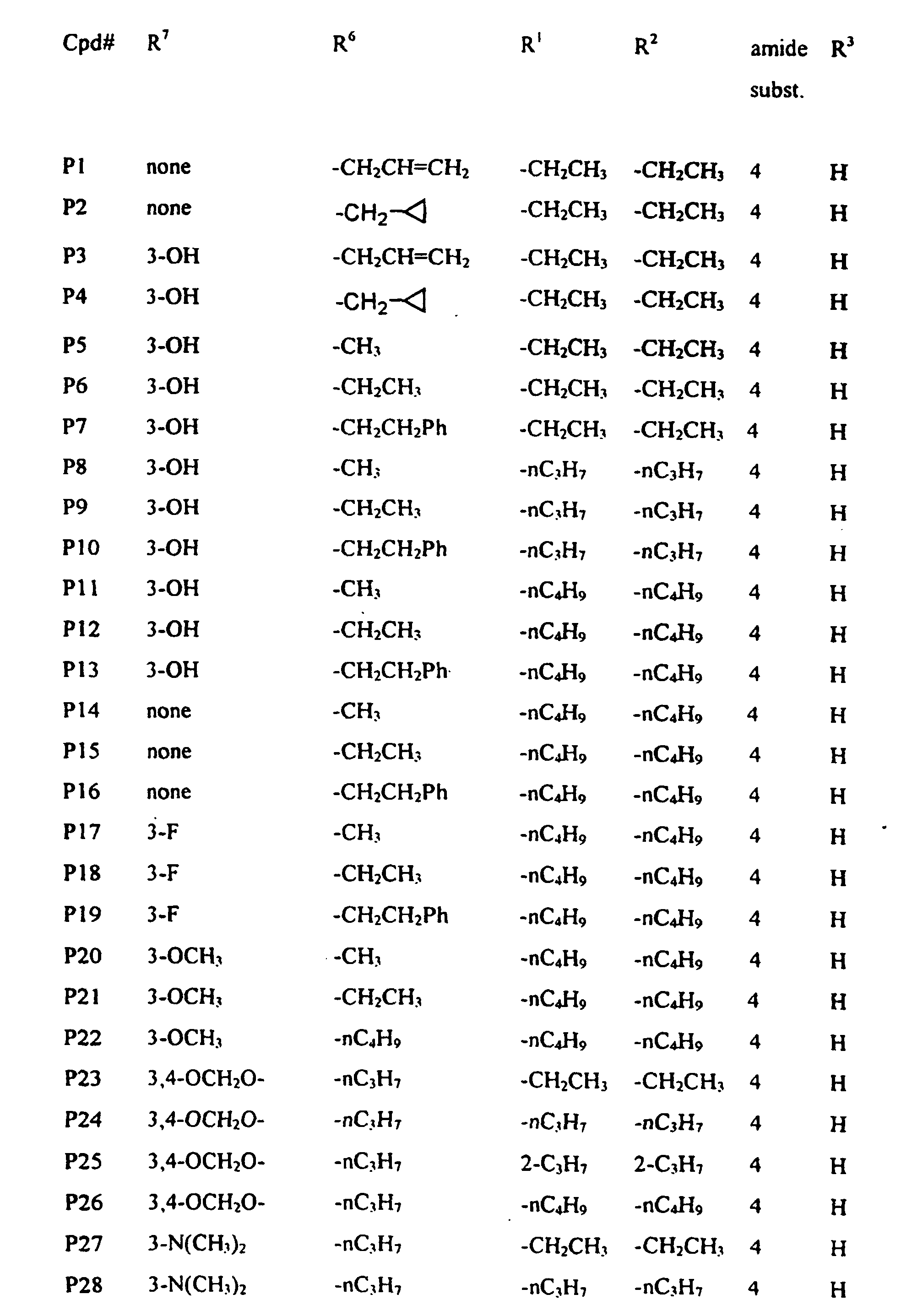

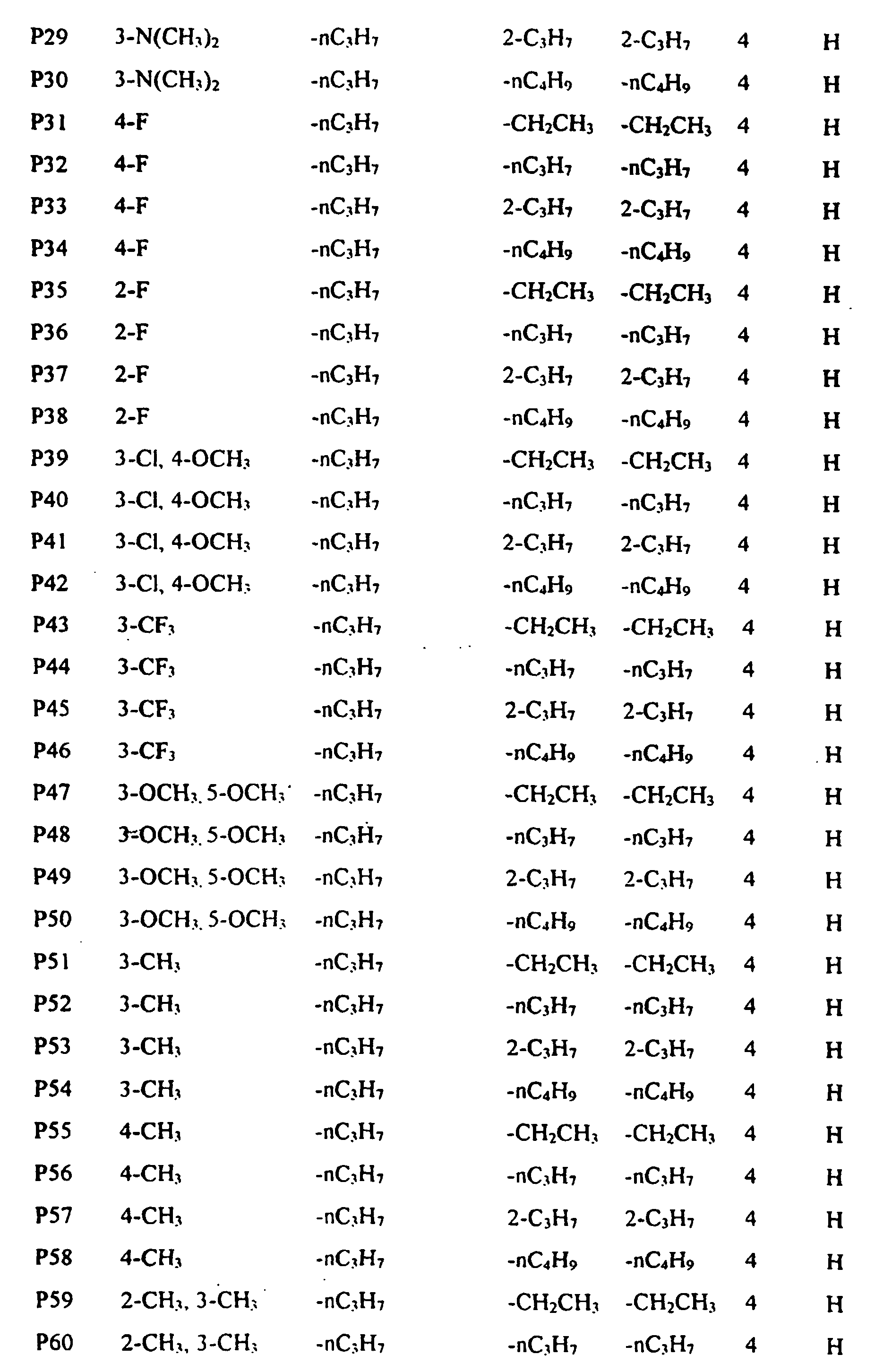

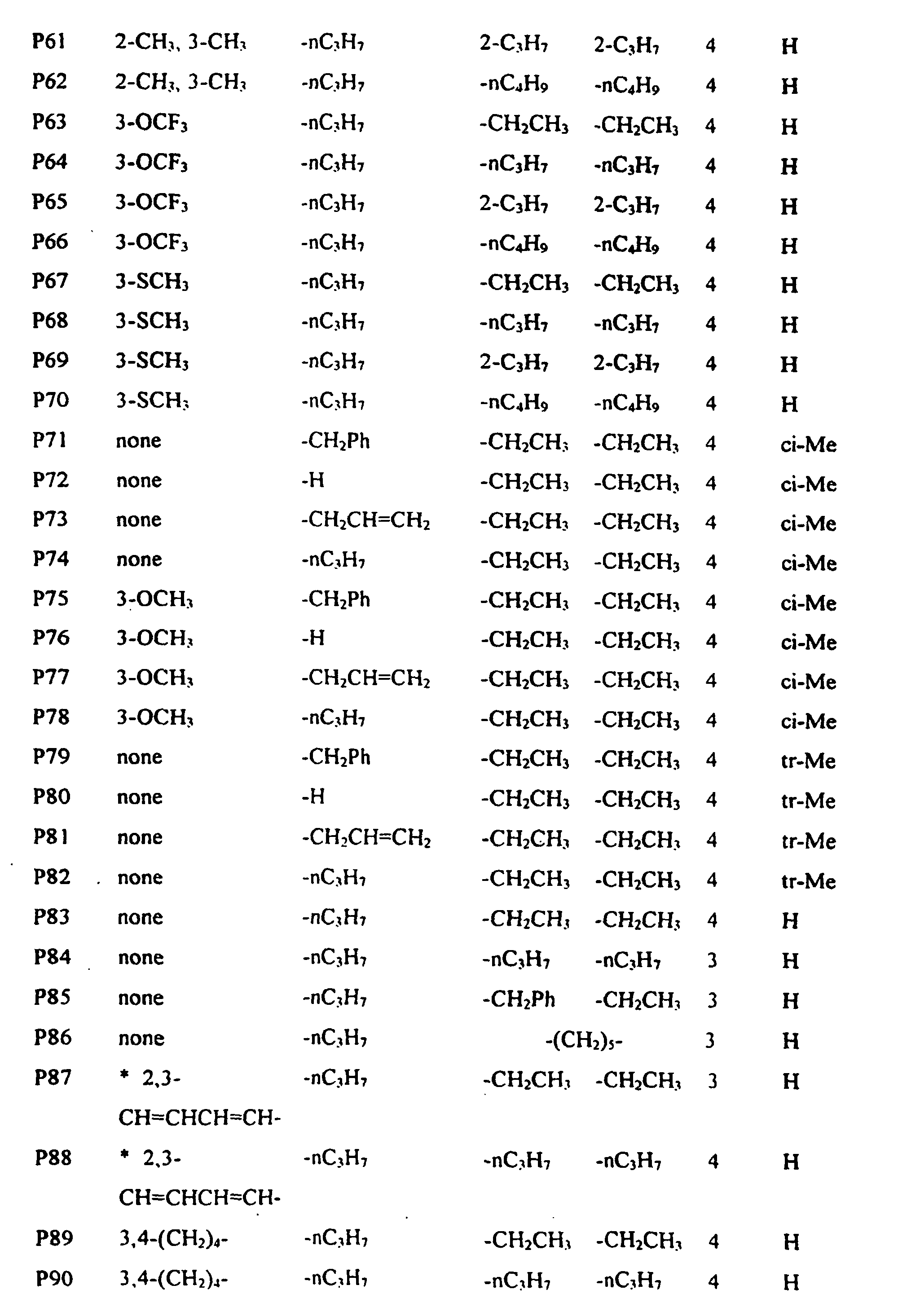

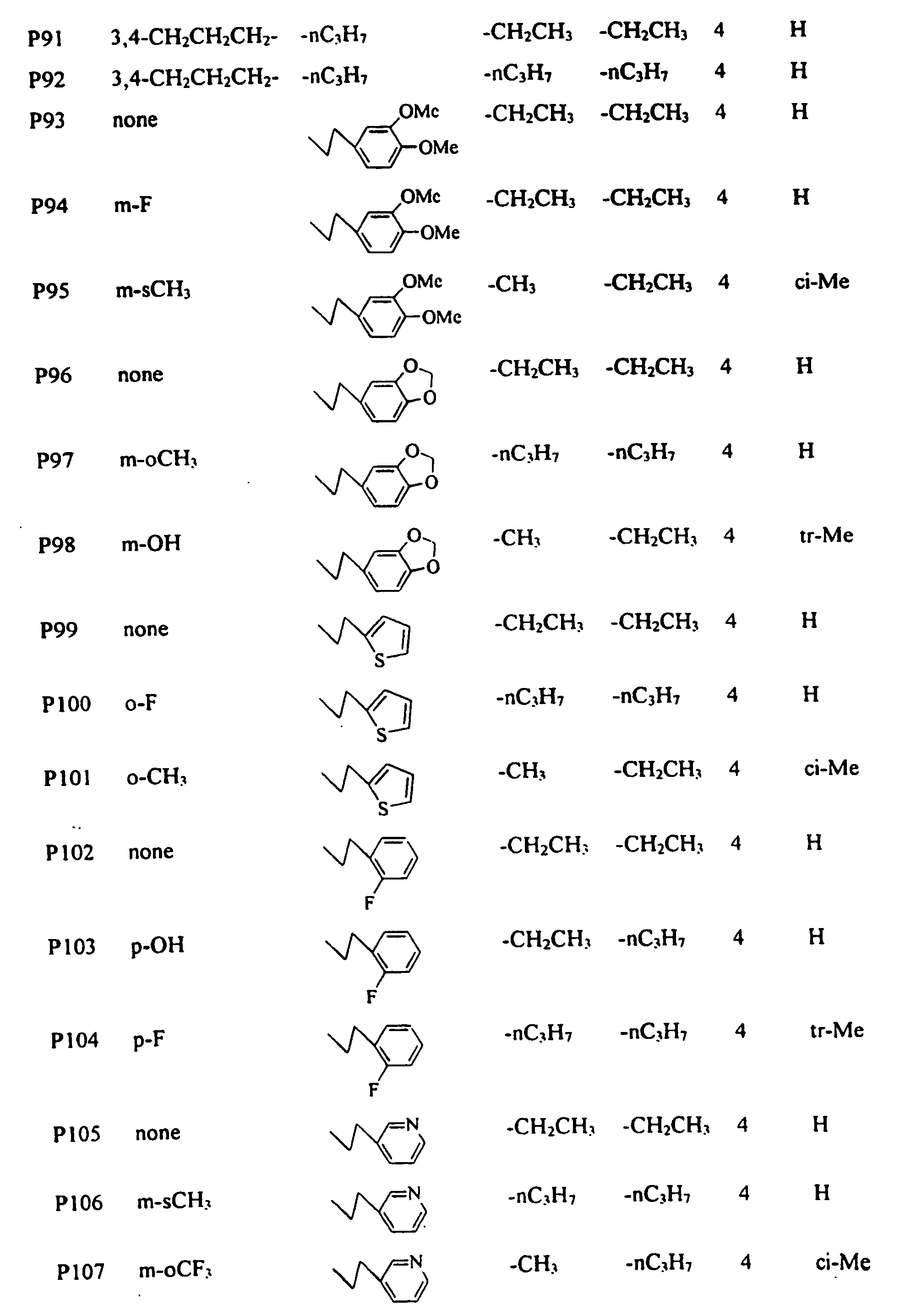

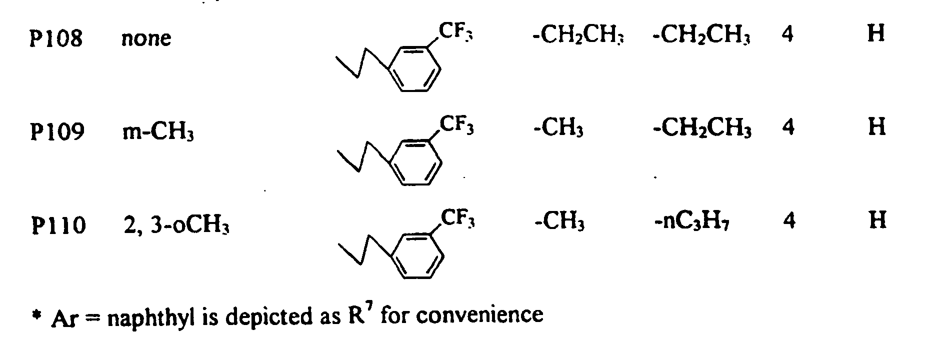

The compounds of the present invention may be used to treat mild to moderately severe pain in warm-blooded animals, such as, humans by administration of an analgesically effective dose. The dosage range would be from about I to 3000 mg, in particular about 10 to 1000 mg or about 25 to 500 mg, of active ingredient I to 4 times per day for an average (70 kg) human although it is apparent that activity of individual compounds of the invention will vary as will the pain being treated. In regards to the use of these compounds as immunosuppressants, antiinflammatory agents, agents for the treatment of mental illness, medicaments for drug and alcohol abuse, agents for treating gastritis and diarrhea, cardiovascular agents, and agents for the treatment of respiratory diseases, a therapeutically effective dose can be determined by persons skilled in the art by use of established animal models. Such dosage would likely fall in the range of from about 1 to 3000 mg of active ingredient 1 to 4 times per day for an average (70 kg) human. Pharmaceutical compositions of the invention comprise the formula (I) compounds as defined above, particularly in admixture with a pharmaceutically-acceptable carrier

To prepare the pharmaceutical compositions of this invention, one or more compounds of the invention or salt thereof as the active ingredient, is intimately admixed with a pharmaceutical carrier according to conventional pharmaceutical compounding techniques, which carrier may take a wide variety of forms depending of the form of preparation desired for administration, e.g., oral or parenteral such as intramuscular. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives. coloring agents and the like, for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. For parenterals, the carrier will usually comprise sterile water, through other ingredients, for example, for purposes such as aiding solubility or for preservation, may be included. Injectable suspensions may also be prepared, in which case appropriate liquid carriers, suspending agents and the like may be employed. The pharmaceutical compositions herein will contain, per dosage unit, e.g., tablet, capsule, powder, injection, teaspoonful and the like, an amount of the active ingredient necessary to deliver an effective dose as described above.

The pharmaceutically acceptable salts referred to above generally take a form in which the nitrogen of the piperidinyl ring is protonated with an inorganic or organic acid. Representative organic or inorganic acids include hydrochlocic, hydrobromic, hydroiodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benezenesulfonic, oxalic, pamoic, 2-naphthalenesulfonic. p-toluenesulfonic, cyclohexanesulfamic, salicylic or saccharic.

The following examples are offered by way of illustration,

### EXAMPLES

### Procedure A

**N-(3-Methoxyphenyl)-1-propyl-4-piperidinamine, I1**. A solution of 4.5 mL (30 mmol) of N-propyl-4-piperidone, 3.4 mL (30 mmol) of *m*-anisidine, and 1.7 mL (30mmol) of glacial acetic acid was stirred in 120 mL of 1.2-dichloroethane (DCE) and 9.49 g (45 mmol) of sodium triacetoxyborohydride was added. The solution was stirred at 25°C for 3h. The solution was washed with NaHCO₃ solution and brine. It was dried and the solvent was evaporated. The *m*-anisidine excess was distilled off in a Kugelrohr at 100°C/0.05 Torr. There was obtained 3.5 g (47% yield) of N-(3-methoxyphenyl)-1-propyl-4-piperidinamine as a solid. MS *m*/*z* = 249 (M⁺+ H). 300 MHz ¹H NMR (CDCl₃) δ 7.1 (t, 1H); 6.15-6.35 (m, 3H); 3.75 (s, 3H); 3.25 (m, 1H); 2.9 (m, 2H); 2.3 (m, 2H); 2.15 (m, 4H); 1.5 (m, 4H); 0.9 (t, 3H). Anal calcd. for C₁₅H₂₄N₂O: C, 72.54; H, 9.74; N, 11.28. Found: C, 72.55; H, 9.51; N, 11.21.

### Example 1

**N,N-Diethyl-4-[3-methoxyphenyl(1-propylpiperidin-4-yl)amino]benzamide Fumarate [1:1],** C1. A solution of 3.5 g (14.1 mmol) of N-(3-methoxyphenyl)-1-propyl-4-piperidinamine, 3.61 g (14.1 mmol) of N,N-diethyl-4-bromobenzamide, 129 mg (0.141 mmol) tris(dibenzylideneacetone)-dipalladium(0) (Pd₂dba₃), 263 mg (0.423 mmol) of (R)-(+)-2,2'-bis(diphenylphosphino)-1.1'-binapthyl (+ BINAP) and 1.89 g (19.7 mmol) of sodium *t*-butoxide in 25 mL of dry toluene was heated at 110°C under Ar in a pressure vessel for 16 h. The mixture was cooled and partitioned between CH₂Cl₂ and H₂O. The organic layer was washed with brine, dried (K₂CO₃) and the solvent was evaporated. The residue was chromatographed on a Biotage Flash 75 unit using CH₂Cl₂:MeOH:NH₄OH, 92:8:0.8 as eluent. There was obtained 3.2 g (53% yield) of N,N-diethyl-4-[3-methoxyphenyl(1-propylpiperidin-4-yl)amino]benzamide as a solid. A fumarate salt was prepared out of 2-PrOH: mp 168-169°C. MS *m*/*z* = 424 (M⁺+ H). 300 MHz ¹H NMR (DMSO-*d*₆) δ 7.3 (t, 1H); 7.2 (d, 2H); 6.8 (d, 1H); 6.6 (m, 4H); 6.5 (s, 2H); 4.0 (m, 1H); 3.75 (s, 3H); 3.3 (q, 4H); 3.1 (d, 2H), 2.4 (m, 4H); 1.9 (d, 2H); 1.4 (m, 4H); 1.1 (t, 6H); 0.8 (t, 3H). Anal calcd. for C₂₆H₃₇N₃O₂•C₄H₄O₄: C, 66.77; H, 7.65; N. 7.78. Found: C, 66.69; H, 7.76; N, 7.68.

### Example 2

**N,N-Diethyl-4-[3-hydroxyphenyl(1-propylpiperidin-4-yl)amino]benzamide Oxalate Hydrate [1.0:0.5:0.25],** C2. A solution of 1.24 g (2.93 mmol) of N,N-diethyl-4-[3-methoxyphenyl(1-propylpiperidin-4-yl)amino]benzamide, C1, in 5 mL of CH₂Cl₂ was cooled to -60°C under Ar and a solution of 17.58 mL (17.58 mmol) of 1.0 M BBr₃ in CH₂Cl₂ was added dropwise. The temperature was allowed to rise to 25°C and the mixture was stirred for 18 h. It was partitioned between NaHCO₃ solution and 25% EtOH in CH₂Cl₂. The organic layer was dried (Na₂SO₄) and the solvent evaporated. The residue was was heated under reflux in 100 mL of saturated NaHCO₃ solution. The solution was cooled and extracted with CH₂Cl₂. The solution was dried (Na₂SO₄) and concentrated to give 1.1 g (92% yield) of N,N-diethyl-4-[3-hydroxyphenyl(1-propylpiperidin-4-yl)amino]benzamide as a gum. An oxalate salt was prepared in CH₃CN, mp 196-197°C. MS *m*/*z* = 410 (M⁺+ H). 300 MHz ¹H NMR (DMSO-*d*₆) δ 7.2(m, 3H); 6.6 (m, 3H); 6.5(d, 1H); 6.4(s, 1H); 4.1(m, 1H); 3.3(q, 4H); 3.2 (m, 2H); 2.6 (m, 4H); 2.1 (d, 2H), 1.5 (m, 4H), 1.1 (t, 6H); 0.8 (t, 3H). Anal calcd. for C₂₅H₃₅N₃O₂•0.5 C₂H₂O₄•0.25 H₂O: C, 68.03, H, 8.01; N, 9.15; H₂O, 0.98. Found: C, 67.73; H, 7.73, N, 9.11; H₂O, 0.46.

### Example 3

**N,N-Diethyl-4-[phenyl(1-propylpiperidin-4-yl)amino]benzamide Fumarate[1:1],** C3. Following the protocol of Procedure A and employing aniline in place of *m*-anisidine, N-phenyl-1-propyl-4-piperidinamine was obtained as a solid: mp 71-72°C. MS *m*/*z* = 217 (M⁺+ H). 300 MHz ¹H NMR (CDCl₃) δ 7.1 (t, 2H); 6.65-6.5 (m, 3H); 3.5 (s, 1H); 3.25 (m, 1H); 3.3 (m, 1H) 2.9 (m, 2H); 2.3 (m, 2H); 2.15 (m, 4H); 1.5 (m, 4H); 0.9 (t, 3H).

Then, following the procedure of Example 1 and employing N-phenyl-1-propyl-4-piperidinamine in place of N-(3-methoxyphenyl)-1-propyl-4-piperidinamine, N,N-diethyl-4-[phenyl(1-propylpiperidin-4-yl)amino]benzamide fumarate was obtained as the product: mp 152-154°C. MS *m*/*z* = 394 (M⁺+ H). 300 MHz ¹H NMR (DMSO-d₆) δ 7.45 (t, 2H); 7.3(t, 1H); 7.2 (d, 2H); 7.05 (d, 2H); 6.55 (d, 2H); 6.5 (s, 2H); 4.0 (m, 1H); 3.3 (q, 4H); 3.1 (d, 2H); 2.4 (m, 4H); 1.9 (d, 2H); 1.4 (m, 4H); 1.1 (t, 6H); 0.8 (t, 3H).

### Example 4

**N-Methyl-N-phenyl-3-[phenyl(1-propylpiperidin-4-yl)amino]benzamide Fumarate[1:1.4],** C4. Following the procedure of Example 1 and employing N-phenyl-1-propyl-4-piperidinamine in place of N-(3-methoxyphenyl)-1-propyl-4-piperidinamine and 3-bromo-N-methylbenzanilide in place of N,N-diethyl-4-bromobenzamide, N,N-diethyl-4-[phenyl(1-propylpiperidin-4-yl)amino]benzamide fumarate [1:1.5] was obtained as the product: mp 190-191°C. MS *m*/*z* = 428 (M⁺+ H). 300 MHz ¹H NMR (DMSO-*d*₆) δ 7.35 (t, 4H); 7.2-6.9 (m, 4H) 6.55 (t, 2H); 6.5 (s, 2H); 3.85 (m, 1H); 3.3 (s, 3H); 2.9 (d, 2H); 2.4 (t, 2H); 2.2 (d, 2H); 1.6 (d, 2H); 1.4 (m, 2H); 1.1 (m, 2H); 0.8 (t, 3H).

### Procedure B

Following the protocol of Procedure A and employing the appropriate aryl amine in place of *m*-anisidine and the requisite N-substituted piperidine in place of N-propylpiperidine, the following N-aryl-1-substituted-4-piperidinamines, I2 - I10, were prepared: 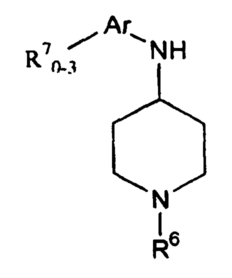 where R⁶ and R⁷ are dependently selected from the groups consisting of:

| Cpd# | R⁷-Ar | R⁶ | MS *m*/*z* (M⁺+ H) |
|---|---|---|---|
| I1 | 3-CH₃O-Ph | 1-Propyl | 249 |
| I2 | 3-Cl-Ph | 1-Propyl | 253 |
| I3 | 2-CH₃O-Ph | 1-Propyl | 249 |
| I4 | 1-Naphthyl | 1-Propyl | 269 |
| I5 | Ph | Methyl | 191 |
| I6 | 3-CH₃O-Ph | Methyl | 221 |
| I7 | 3-F-Ph | Methyl | 209 |
| I8 | Ph | Ethyl | 205 |
| I9 | 3-F-Ph | Ethyl | 223 |
| I10 | Ph | Benzyl | 267 |

### Example 5

Following the procedure of Example 1 and employing the appropriate N-aryl-4-piperidinamine in place of N-(3-methoxyphenyl)-1-propyl-4-piperidinamine and the requisite 4-bromobenzamide in place of N,N-diethyl-4-bromobenzamide, the following compounds C5 - C45 were obtained: 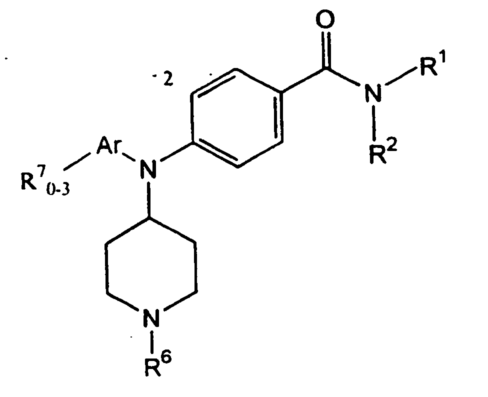 where R¹, R², R⁶ and R⁷-Ar are dependently selected from the groups consisting of:

| Cpd# | R⁷-Ar | R⁶ | R¹ | R² | MS | Isol'n |
|---|---|---|---|---|---|---|
| C5 | 3-Cl-Ph | 1-Propyl | Ethyl | Ethyl | 428 | A |
| C6 | 3-Cl-Ph | 1-Propyl | -(CH₂)₄- | | 426 | A |
| C7 | 2-CH₃O-Ph | 1-Propyl | Ethyl | Ethyl | 424 | A |
| C8 | 1-Naphthyl | 1-Propyl | Ethyl | Ethyl | 416 | A |
| C9 | 1-Naphthyl | 1-Propyl | -(CH₂)₄- | | 456 | A |
| C10 | Ph | Methyl | Ethyl | Ethyl | 366 | B |
| C11 | Ph | Methyl | 1-Propyl | 1-Propyl | 394 | B |
| C12 | Ph | Methyl | Methyl | Ethyl | 352 | B |
| C13 | Ph | Methyl | 2-Propyl | 2-Propyl | 394 | B |
| C14 | Ph | Methyl | -(CH₂)₂-O-(CH₂)₂- | | 380 | B |
| C15 | Ph | Methyl | -(CH₂)₅- | | 378 | B |
| C16 | Ph | Methyl | -(CH₂)₄- | | 364 | B |
| C17 | 3-CH₃O-Ph | Methyl | Ethyl | Ethyl | 396 | B |
| C18 | 3-CH₃O-Ph | Methyl | 1-Propyl | 1-Propyl | 424 | B |
| C19 | 3-CH₃O-Ph | Methyl | Methyl | Ethyl | 382 | B |
| C20 | 3-CH₃O-Ph | Methyl | 2-Propyl | 2-Propyl | 424 | B |
| C21 | 3-CH₃O-Ph | Methyl | -(CH₂)₂-O-(CH₂)₂- | | 410 | B |
| C22 | 3-CH₃O-Ph | Methyl | -(CH₂)₅- | | 408 | B |
| C23 | 3-CH₃O-Ph | Methyl | -(CH₂)₄- | | 394 | B |
| C24 | 3-F-Ph | Methyl | Ethyl | Ethyl | 384 | B |
| C25 | - 3-F-Ph | Methyl | 1-Propyl | 1-Propyl | 412 | B |
| C26 | 3-F-Ph | Methyl | Methyl | Ethyl | 370 | B |
| C27 | 3-F-Ph | Methyl | 2-Propyl | 2-Propyl | 412 | B |
| C28 | 3-F-Ph | Methyl | -(CH₂)₂-O-(CH₂)₂- | | 398 | B |
| C29 | 3-F-Ph | Methyl | -(CH₂)₅- | | 396 | B |
| C30 | 3-F-Ph | Methyl | -(CH₂)₄- | | 382 | B |
| C31 | Ph | Ethyl | Ethyl | Ethyl | 380 | B |
| C32 | Ph | Ethyl | 1-Propyl | 1-Propyl | 408 | B |
| C33 | Ph | Ethyl | Methyl | Ethyl | 366 | B |
| C34 | Ph | Ethyl | 2-Propyl | 2-Propyl | 408 | B |
| C35 | Ph | Ethyl | -(CH₂)₂-O-(CH₂)₂- | | 394 | B |
| C36 | Ph | Ethyl | -(CH₂)₅- | | 392 | B |
| C37 | Ph | Ethyl | -(CH₂)₄- | | 378 | B |
| C38 | 3-F-Ph | Ethyl | Ethyl | Ethyl | 398 | B |
| C39 | 3-F-Ph | Ethyl | 1-Propyl | 1-Propyl | 426 | B |
| C40 | 3-F-Ph | Ethyl | Methyl | Ethyl | 384 | B |
| C41 | 3-F-Ph | Ethyl | 2-Propyl | 2-Propyl | 426 | B |
| C42 | 3-F-Ph | Ethyl | -(CH₂)₂-O-(CH₂)₂- | | 412 | B |
| C43 | 3-F-Ph | Ethyl | -(CH₂)₅- | | 410 | B |
| C44 | 3-F-Ph | Ethyl | -(CH₂)₄- | | 396 | B |
| C45 | Ph | Benzyl | Ethyl | Ethyl | 442 | A |

Isolation, Method A: chromatography on SiO₂ with CH₂Cl₂ \ MeOH \ NH₄OH; 95\5\0.5.
Method B: Reverse phase HPLC on YMC J' sphere H80 (20/80 MeCN/0.1% aq. TFA to 90/10 MeCN/0.1% aq. TFA)

### Example 6

**N,N-Diethyl-4-[3-acetoxyphenyl(1-propylpiperidin-4-yl)amino]benzamide Hydrochloride[1:1],** C46. A solution of 0.96 g (2.3 mmol) of N,N-diethyl-4-[3-hydroxyphenyl(1-propylpiperidin-4-yl)amino]benzamide was stirred in 20 mL of CH₂Cl₂ and 0.17 mL of acetyl chloride was added. The mixture was stirred for 2 h. The solvent was evaporated and the residue recrystallized from 2-PrOH to give 0.7g (62% yield) of the title compound as a white crystalline solid: mp 218-219°C. MS *m*/*z* = 452 (M⁺+ H). 300MHz ¹H NMR (DMSO-*d*₆) δ 7.3 (d 2H); 7.2 (m, 1H);6.9 (d, 2H);6.7 (m, 2H);4.1 (t, 3H); 3.6 (d, 2H); 3.4 (m, 4H); 2.8 (m, 4H); 2.4(m, 2H); 2.2(s, 3H) 2.1 (d, 2H); 1.9 (m, 2H); 1.2 (t, 6H), 1.0 (t, 3H). Anal calcd. for C₂₇H₃₇N₃O₃•HCl: C, 66.45; H, 7.45; N, 8.61. Found: C, 66.07; H, 7.83; N, 8.32.

### Biological Testing

Delta- and mu-opioid receptor binding of the above compounds was determined according to the following procedures and the following results were obtained.

### 1) High Throughput Screening Assay for Delta-Opioid Receptor Binding

Materials: This is a receptor based screen to detect the competitive binding of test compounds at the opioid delta receptor against the radioligand, [³H]bremazocine (S.A.=25.5 Ci/mmol, Dupont/NEN, Cambridge, Massachusetts). The receptor is a cloned human cDNA expressed in mammalian CHO cells. Membranes prepared from these cells are purchased from Receptor Biology, Baltimore, MD. The reaction buffer is composed as follows: HEPES (50 mM final), MgCl•6H₂O (5 mM final), o-phenanthroline (20 mg/l), aprotinin (10 mg/l), Pefabloc SC (250 mg/l), leupeptin (0.5 mg/l), pepstatin A (0.7 mg/l), trypsin inhibitor (25 mg/l), chymostatin (10 mg/l), pH = 7.2. Naloxone, 10 uM, is used to define non-specific binding. The assay employs filtration to capture receptor and bound ligand.
Procedure: The receptor (membrane) preparation (28 ug protein) is allowed to incubate with the opioid receptor radioligand ([³H]bremazocine, 2.4 nM) in 96-well plates until equilibrium is reached (>2 hr). Following incubation with the radioligand at 23°C, the well contents are filtered onto 96-well Whatman GF/C filter plates using a Packard cell harvester. Radioligand bound to the receptor also remains on the filter. The filters are rinsed three times with 0.5 mL of physiological saline (0.9% NaCl) to remove any unbound radioligand from the filters. Filters are dried and scintillation fluid is then added to the filters which emits light in proportion to the amount of radioactivity on the filter which is determined using an Packard Topcount scintillation counter.
Principle: Unknown drugs included in the incubation which bind to the same receptor as the radioligand will compete for the receptor and reduce the amount of radioligand which binds to the receptor. This is detected as a decreased scintillation signal from that particular incubation. The better an unknown competes for the receptor, the larger the observed decrease in radioligand bound to the receptor; thus the assay is in the format of an inhibition study. Data are reported as percent inhibition of control binding.
Results: The following compounds were tested with the following results.

| Cpd# | %1 @ 25 µM | Cpd# | %1 @ 25 µM |
|---|---|---|---|
| C1 | 97 | C11 | 101 |
| C3 | 94 | C12 | 95 |
| C4 | 41 | C13 | 100 |
| C5 | 102 | C14 | 93 |
| C6 | 101 | C15 | 68 |
| C7 | 100 | C16 | 81 |
| C8 | 69 | C17 | 98 |
| C9 | 103 | C18 | 100 |
| C10 | 100 | C19 | 95 |
| C20 | 98 | C34 | 100 |
| C21 | 70 | C35 | 64 |
| C22 | 89 | C36 | 92 |
| C23 | 91 | C37 | 88 |
| C24 | 97 | C38 | 98 |
| C25 | 100 | C39 | 101 |
| C26 | 96 | C40 | 97 |
| C27 | 97 | C41 | 88 |
| C28 | 77 | C42 | 64 |
| C29 | 85 | C43 | 89 |
| C30 | 85 | C44 | 87 |
| C31 | 100 | C45 | 99 |
| C32 | 100 | C46 | 100 |
| C33 | 98 | | |

### 2) Manual Tissue Screening Assay for Delta- and Mu-Opioid Receptor Binding

### A) Rat Brain δ-Opioid Receptor Binding Assay

Procedure: Male, Wistar rats (150-250 g, VAF, Charles River, Kingston, NY) are killed by cervical dislocation, and their brains removed and placed immediately in ice cold Tris HCl buffer (50 mM, pH 7.4). The forebrains are separated from the remainder of the brain by a coronal transection, beginning dorsally at the colliculi and passing ventrally through the midbrain-pontine junction. After dissection, the forebrains are homogenized in Tris buffer in a Teflon®-glass homogenizer. The homogenate is diluted to a concentration of 1 g of forebrain tissue per 100 mL Tris buffer and centrifuged at 39,000 X G for 10 min. The pellet is resuspended in the same volume of Tris buffer with several brief pulses from a Polytron homogenizer. This particulate preparation is used for the δ-opioid binding assays. Following incubation with the δ-selective peptide ligand [³H]DPDPE at 25°C, the tube contents filtered through Whatman GF/B filter sheets on a Brandel cell harvester. The tubes and filters are rinsed three times with 4 mL of 10 mM HEPES (pH7.4), and the radioactivity associated with the filter circles determined using Formula 989 scintillation fluid (New England Nuclear, Boston, MA) in a scintillation counter.
Analysis: The data are used to calculate either the % inhibition compared to control binding (when only a single concentration of test compound is evaluated) or a Kᵢ value (when a range of concentrations is tested).$\text{% inhibition is calculated as: 1-(test compound dpm-nonspecific dpm)/(total dpm-} \text{nonspecific dpm)*100}$ Kᵢ values are calculated using the LIGAND (Munson, P.J. and Rodbard, D., Anal. Biochem. 107: 220-239, 1980) data analysis program.

### B) Rat Brain µ-Opioid Receptor Binding Assay

Procedure: Male, Wistar rats (150-250 g, VAF, Charles River, Kingston, NY) are killed by cervical dislocation, and their brains removed and placed immediately in ice cold Tris HCl buffer (50 mM, pH 7.4). The forebrains are separated from the remainder of the brain by a coronal transection, beginning dorsally at the colliculi and passing ventrally through the midbrain-pontine junction. After dissection, the forebrains are homogenized in Tris buffer in a Teflon®-glass homogenizer. The homogenate is diluted to a concentration of 1 g of forebrain tissue per 100 mL Tris buffer and centrifuged at 39,000 X G for 10 min. The pellet is resuspended in the same volume of Tris buffer with several brief pulses from a Polytron homogenizer. This particulate preparation is used for the µ-opioid binding assays. Following incubation with the m- selective peptide ligand [³H]DAMGO at 25⁰C, the tube contents are filtered through Whatman GF/B filter sheets on a Brandel cell harvester. The tubes and filters are rinsed three times with 4 mL of 10 mM HEPES (pH7.4), and the radioactivity associated with the filter circles determined using Formula 989 scintillation fluid (New England Nuclear, Boston, MA) in a scintillation counter.
Analysis: The data are used to calculate either the % inhibition compared to control binding (when only a single concentration of test compound is evaluated) or a Kᵢ value (when a range of concentrations is tested).$\text{% inhibition is calculated as: 1-(test compound dpm-nonspecific dpm)/(total dpm-} \text{nonspecific dpm)*100}$ Kᵢ values are calculated using the LIGAND (Munson, P.J. and Rodbard, D., Anal. Biochem. 107: 220-239, 1980) data analysis program.
Results: N,N-Diethyl-4-[phenyl(1-propylpiperidin-4-yl)amino]benzamide fumarate, C4, exhibited a Kᵢ of 25 nM in binding to the δ-opioid receptor and a Kᵢ of 153 nM in binding to the µ-opioid receptor. N,N-Diethyl-4-[3-hydroxyphenyl(1-propylpiperidin-4-yl)amino]benzamide fumarate. C2, exhibited a Kᵢ of 0.83 nM in binding to the δ-opioid receptor and a Kᵢ of 2,762 nM in binding to the µ-opioid receptor.

The activity of compounds of the invention as analgesics may be demonstrated by the mouse acetylcholine-bromide induced constriction assay as described below:

### C) Mouse Acetylcholine Bromide-Induced Abdominal Constriction Assay

Procedure: The mouse acetylcholine-induced abdominal constiction assay, as described by Collier et al. in *Brit. J. Pharmacol. Chem. Ther.,* 32: 295-310, 1968, with minor modifications was used to assess analgesic potency of the compounds of formula (I). The test drugs or appropriate vehicles were administered orally (p.o.) and 30 minutes later the animal received an intraperitoneal (i.p.) injection of 5.5 mg/kg acetylcholine bromide (Matheson, Coleman and Bell, East Rutherford, NJ). The mice were then placed in groups of three into glass bell jars and observed for a ten minute observation period for the occurrence of an abdominal constriction response (defined as a wave of constriction and elongation passing caudally along the abdominal wall, accompanied by a twisting of the trunk and followed by extension of the hind limbs). The percent inhibition of this response to a nociceptive stimulus (equated to % analgesia) was calculated as follows: The % inhibition of response, i.e., % analgesia is equal to the difference between the No. of control animal responses and the No. of drug-treated animal responses times 100 divided by the No. of control animals responding.
Results: N,N-Diethyl-4-[phenyl(1-propylpiperidin-4-yl)amino]benzamide fumarate, C4. exhibited an ED₅₀ of 4.2 µmol/kg in this assay.

## Claims

1. A compound which binds to the delta-opioid receptor of the general formula: 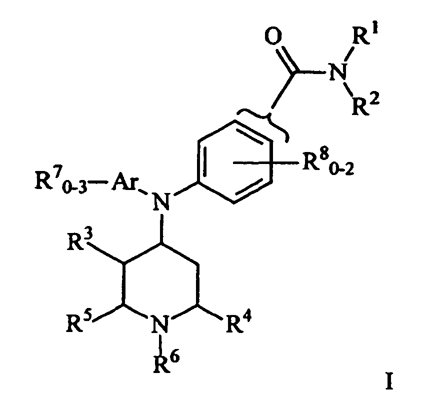 where
Ar is phenyl, 1-naphthyl or 2-naphthyl, each optionally substituted with 1 to 3 R⁷;
R¹ and R² are independently selected from the group consisting of hydrogen, C_{1 8}alkyl; phenyl, optionally mono-, di-, or tri-substituted with halo, C₁₋₆alkyl, C₁₋₆alkoxy or trifluoromethyl; or benzyl, optionally mono-, di-, or tri-substituted with halo, C₁₋₆alkyl, C₁₋₆alkoxy or trifluoromethyl, or alternatively, R¹ and R² are taken together with their N of attachment to form a ring which is selected from the group consisting of pyrrolidinyl, morpholinyl, piperidinyl and hexamethyleneiminyl, each said ring optionally substituted with 1 to 4 methyl groups;
R³ is selected from hydrogen and C₁₋₄alkyl;
R⁴ and R⁵ are hydrogen;
R⁶ is selected from the group consisting of hydrogen; C₁₋₈alkyl; C₃₋₆cycloalkylC₁₋₃alkyl; C₃₋₆alkenyl; C₁₋₆alkoxyC₁₋₃alkyl; and phenylC₁₋₄alkyl, where the phenyl is optionally mono-, di-, or tri-substituted with R⁷;
R⁷ is independently selected from the group consisting of hydroxy, halo, C₁₋₃alkyl, C₁₋₃alkoxy, C₁₋₃acyl, C₁₋₃acyloxy, cyano, amino, C₁₋₃acylamino, C₁₋₃alkylamino, di(C₁₋₃alkyl)amino, C₁₋₃alkylthio, C₁₋₃alkylsulfonyl, trifluoromethyl and trifluoromethoxy, and two R⁷ can together form a single moiety selected from the group consisting of -(CH₂)₃₋₅- and -O(CH₂)₁₋₃O- attached to adjacent carbon atoms of Ar; and
R⁸ is independently selected from the group consisting of halo, C₁₋₆alkyl, C₁₋₆alkoxy and trifluoromethyl;
wherein the compounds of the formulae: 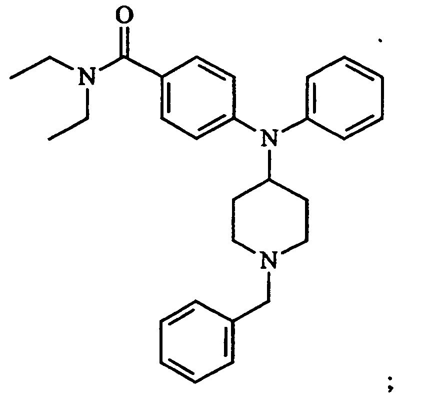 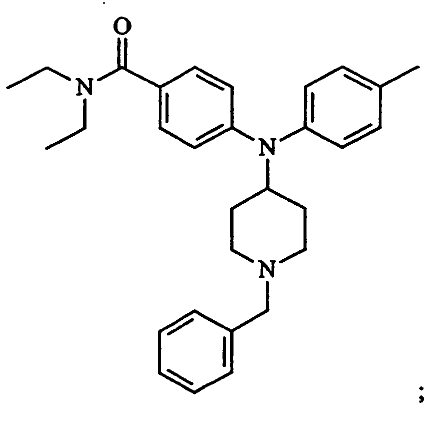 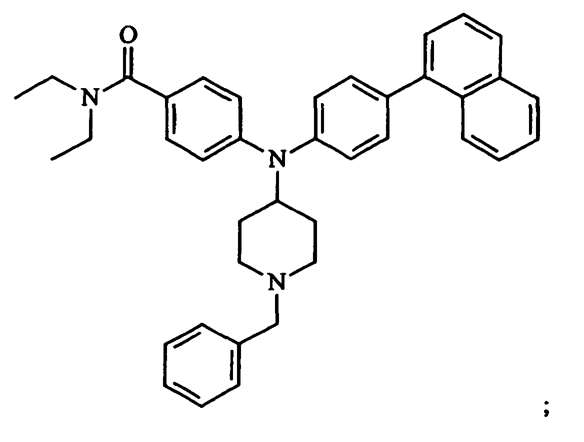 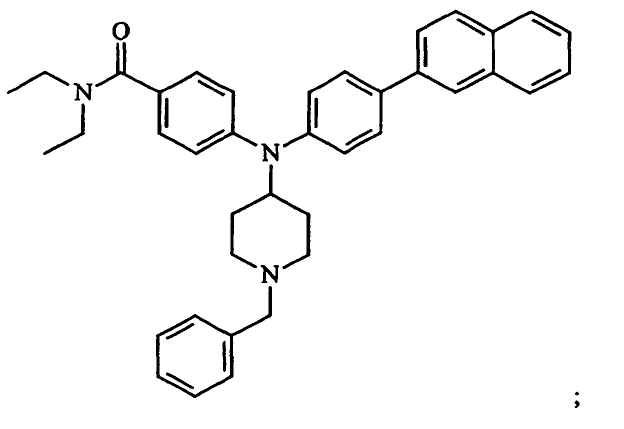 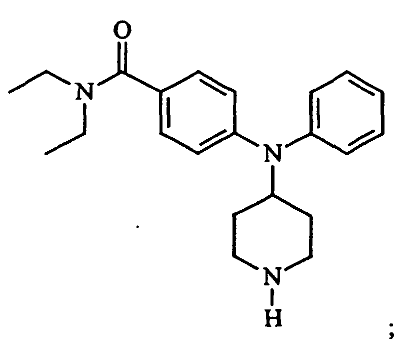 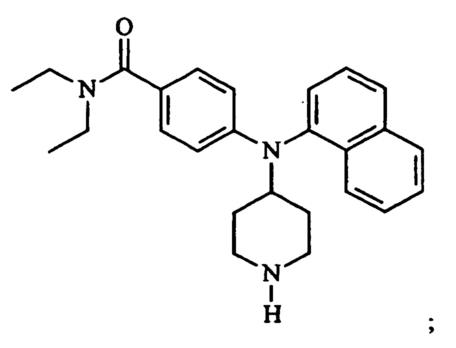 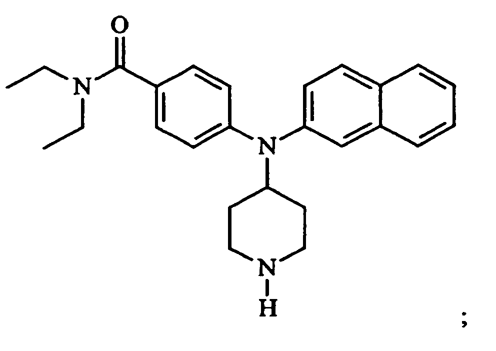 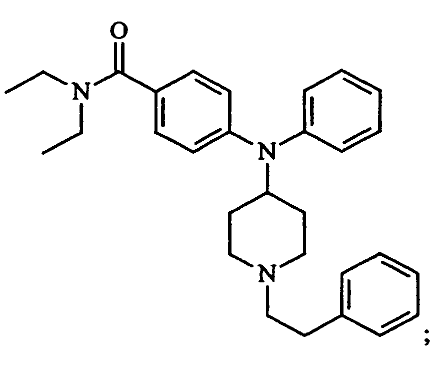 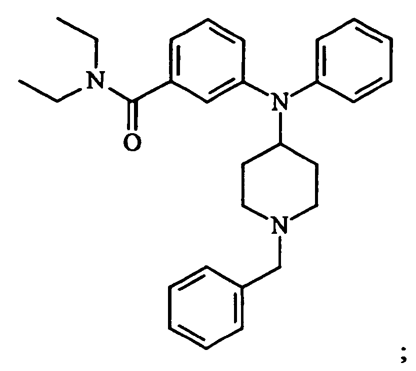 and 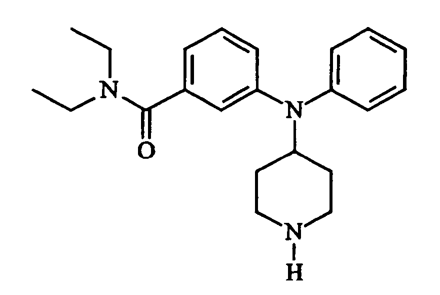 are disclaimed.

2. The compound of claim 1 wherein Ar is phenyl.

3. The compound of claim 1 wherein R¹ and R² are independently selected from the group consisting of hydrogen, methyl, ethyl, n-propyl, i-propyl, n-butyl, t-butyl, i-butyl, phenyl, p-chlorophenyl, p-fluorophenyl, p-methylphenyl, p-trifluoromethylphenyl, benzyl, p-chlorobenzyl, p-fluorobenzyl, p-methylbenzyl and p-trifluoromethylbenzyl, or alternatively, R¹ and R² are taken together with their N of attachment to form a ring which is selected from the group consisting of pyrrolidinyl and piperidinyl.

4. The compound of claim 1 wherein R³ is selected from hydrogen, methyl, ethyl, n-propyl, i-propyl and t-butyl.

5. The compound of claim 1 wherein R⁶ are selected from the group consisting of hydrogen, methyl, ethyl, n-propyl, i-propyl, n-butyl, i-butyl, t-butyl, cyclopropylmethyl, ethenyl, allyl, methoxymethyl, benzyl, p-chlorobenzyl, p-fluorobenzyl, p-methylbenzyl, p-trifluoromethylbenzyl, p-aminobenzyl, and phenylCH₂CH₂-.

6. The compound of claim 1 wherein R⁷ are independently selected from the group consisting of hydroxy, chloro, bromo, fluoro, methyl, ethyl, n-propyl, i-propyl, n-butyl, i-butyl, t-butyl, methoxy, ethoxy, formyl, acyl, acetoxy, cyano, amino, methylamido, methylamino, N,N-dimethylamino, methylthio, methylsulfonyl, trifluoromethoxy and trifluoromethyl, and preferred moieties where two R⁷ together form a single moiety are selected from the group consisting of propylene, butylene and -OCH₂O-.

7. The compound of claim 1 wherein R⁸ are independently selected from the group consisting of chloro, bromo, fluoro, methyl, ethyl, n-propyl, i-propyl, t-butyl, methoxy, ethoxy and trifluoromethyl.

8. The compound of claim 1 having the general structure: 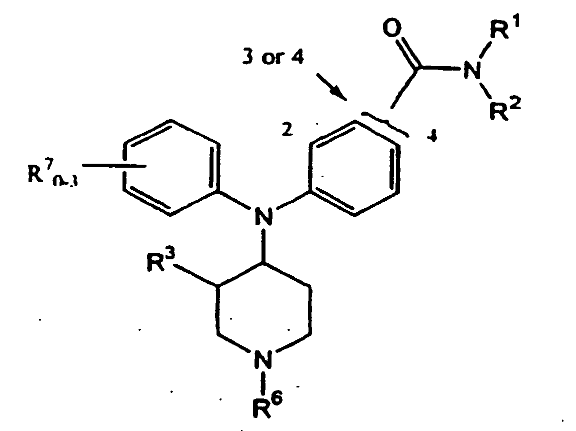 where R¹, R², R³, R⁶ and R⁷ are dependently selected from the groups consisting of:

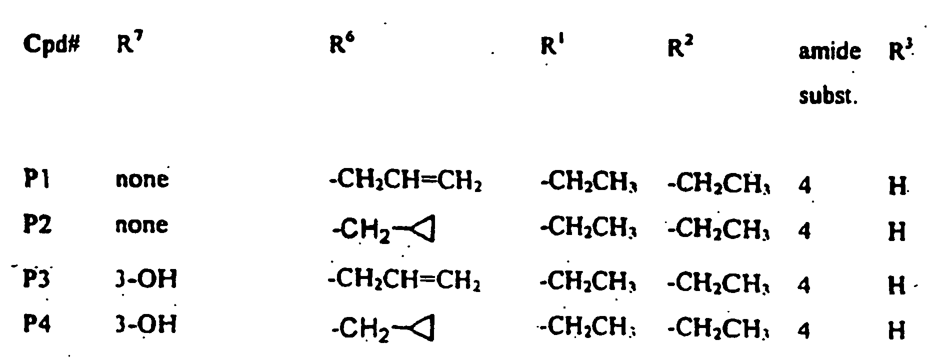

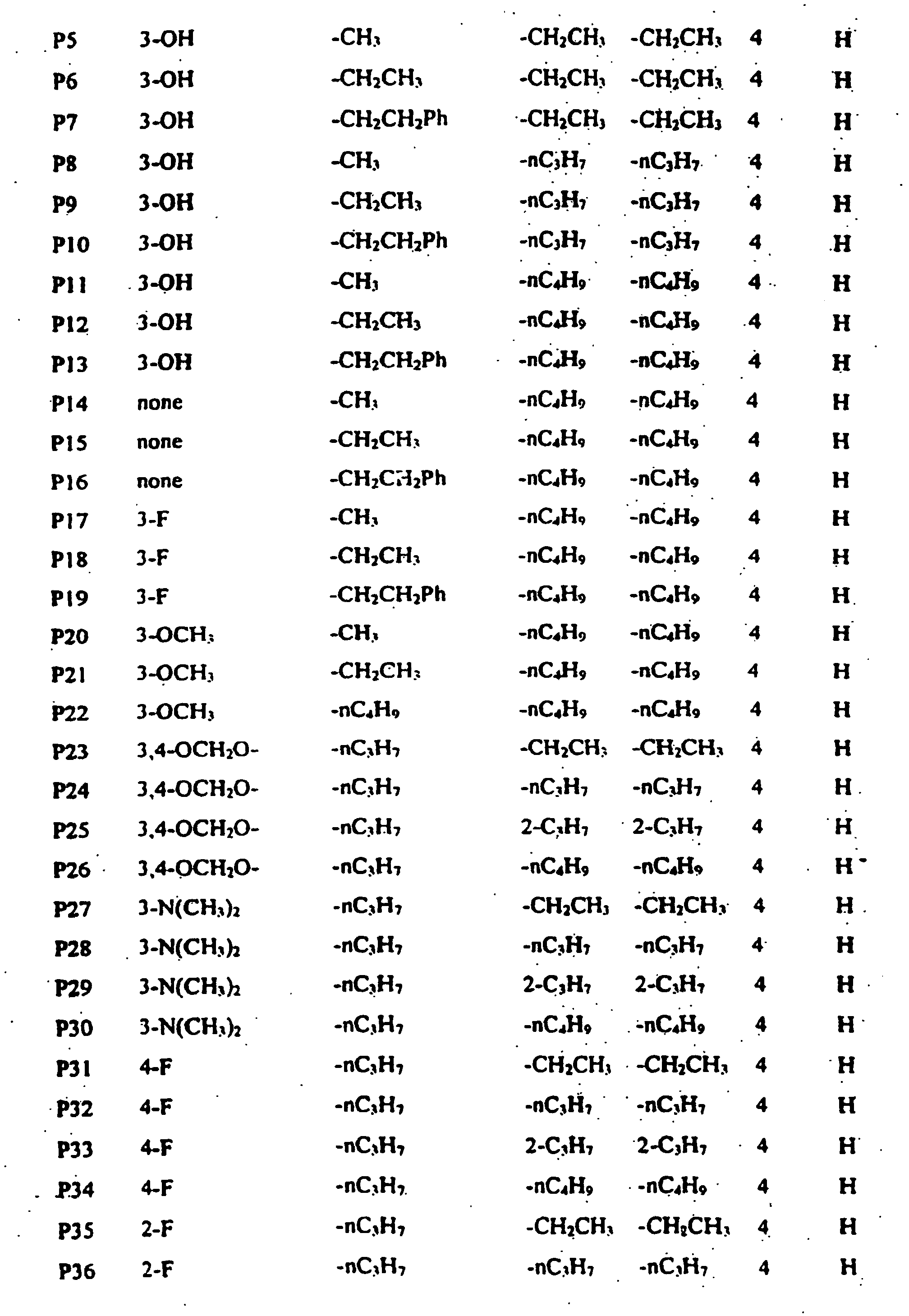

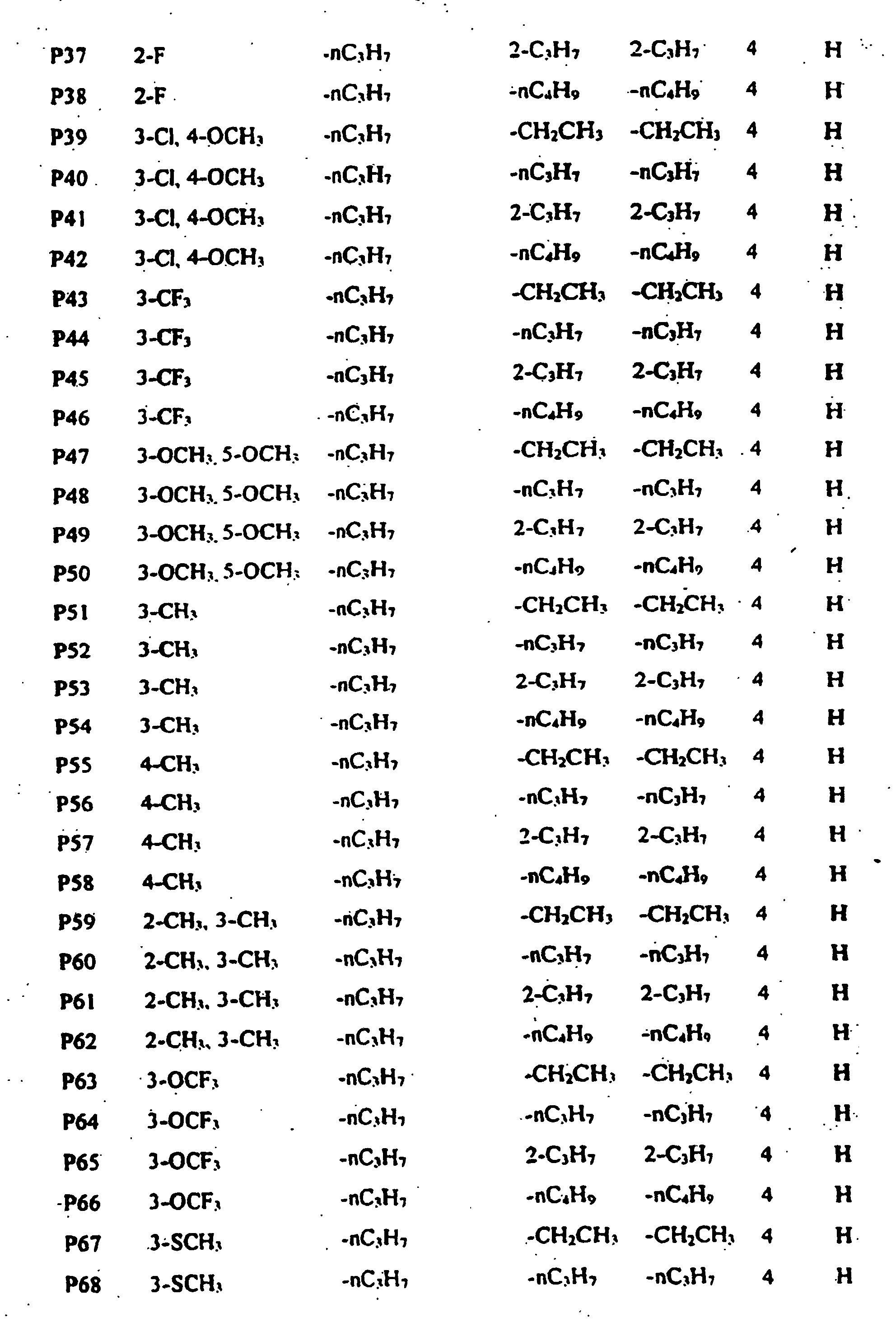

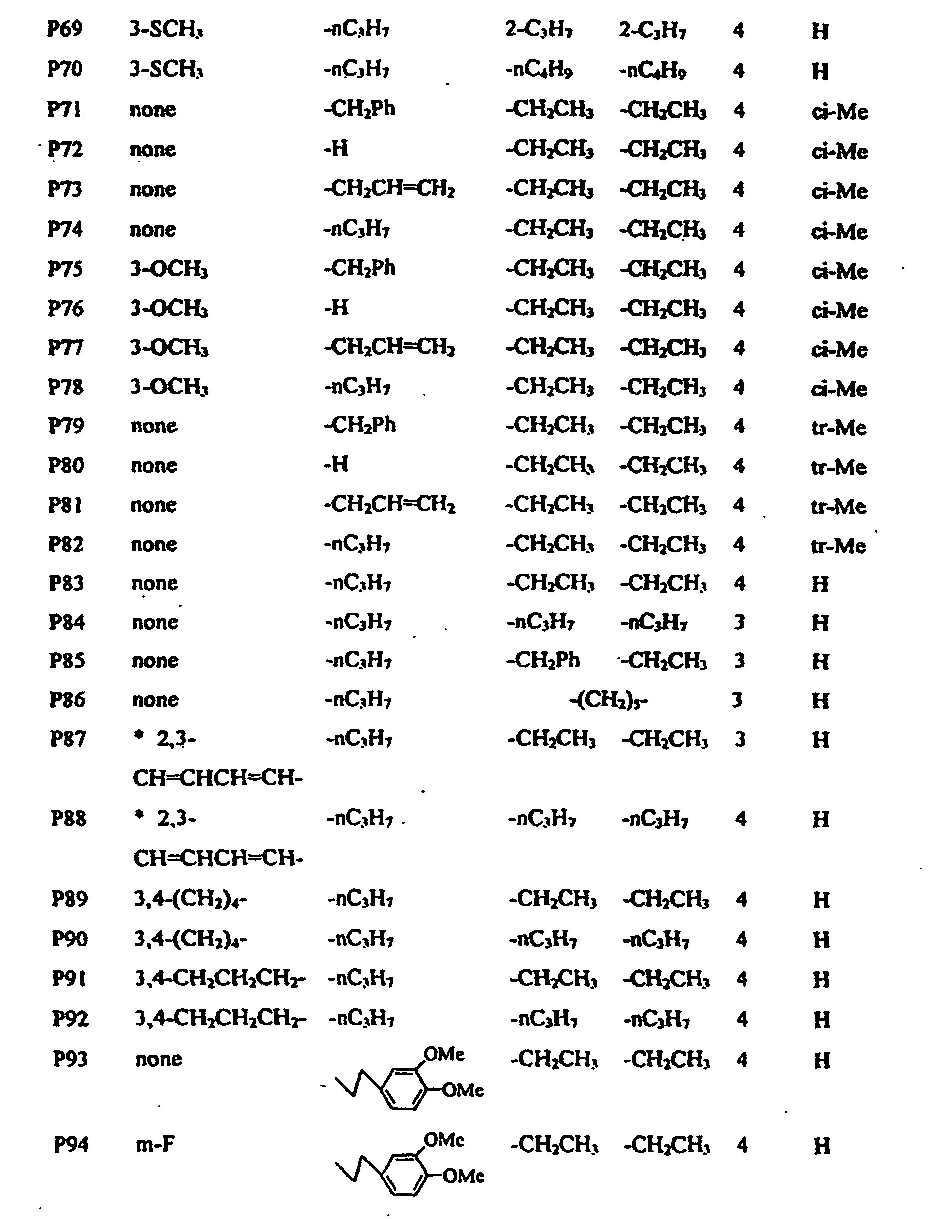

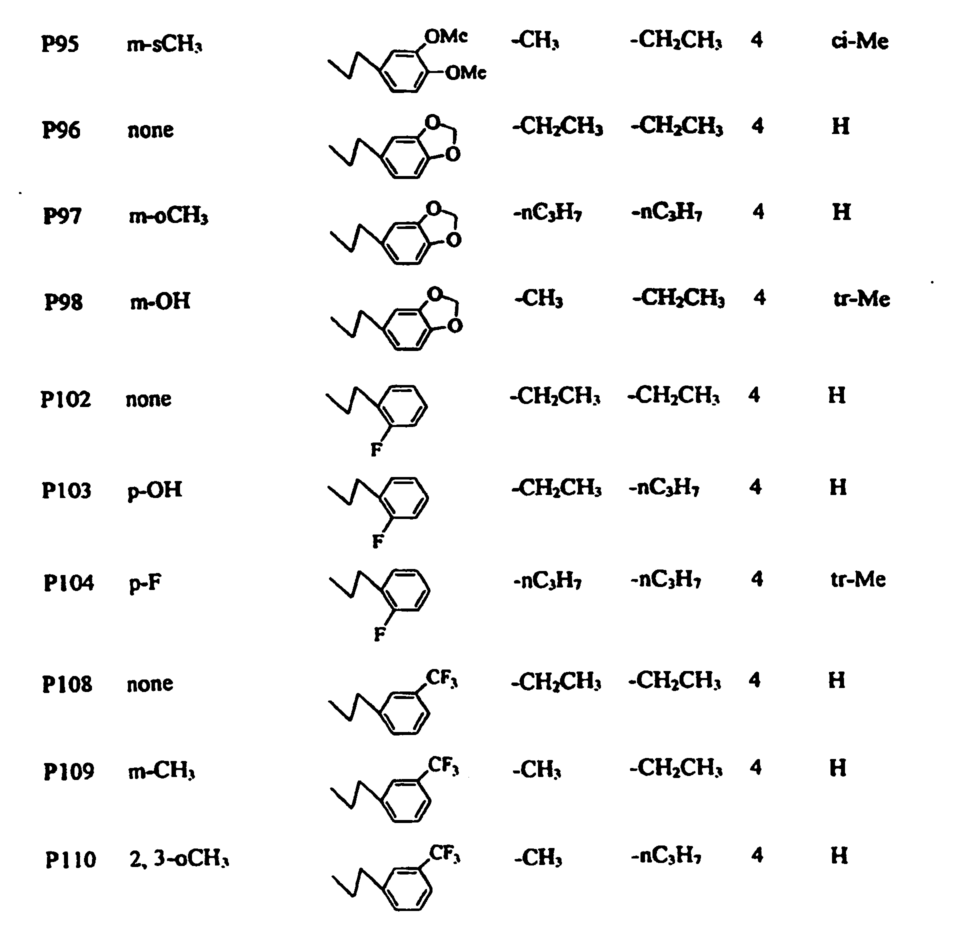

9. The compound of claim 1 having the general structure: 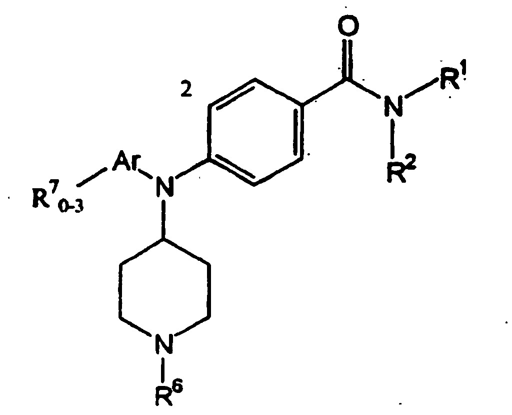 where R¹, R², R⁶ and R⁷-Ar are dependently selected from the groups consisting of:
| Cpd# | R⁷-Ar | R⁶ | R¹ | R² |
|---|---|---|---|---|
| C1 | 3-CH₃O-Ph | 1-Propyl | Ethyl | Ethyl |
| C2 | 3-HO-Ph | 1-Propyl | Ethyl | Ethyl |
| C3 | Ph | 1-Propyl | Ethyl | Ethyl |
| C4 | Ph | 1-Propyl | Methyl | Ph |
| C5 | 3-Cl-Ph | 1-Propyl | Ethyl | Ethyl |
| C6 | 3-Cl-Ph | 1-Propyl | -(CH₂)₄- | |
| C7 | 2-CH₃O-Ph | 1-Propyl | Ethyl | Ethyl |
| C8 | 1-Naphthyl | 1-Propyl | Ethyl | Ethyl |
| C9 | 1-Naphthyl | 1-Propyl | -(CH₂)₄- | |
| C10 | Ph | Methyl | Ethyl | Ethyl |
| C11 | Ph | Methyl | 1-Propyl | 1-Propyl |
| C12 | Ph | Methyl | Methyl. | Ethyl |
| C13 | Ph | Methyl | 2-Propyl | 2-Propyl |
| C14 | Ph | Methyl | -(CH₂)₂-O-(CH₂)₂- | |
| C15 | Ph | Methyl | -(CH₂)₅- | |
| C16 | Ph | Methyl | -(CH₂)₄- | |
| C17 | 3-CH₃O-Ph | Methyl | Ethyl | Ethyl |
| C18 | 3-CH₃O-Ph | Methyl | 1-Propyl | 1-Propyl |
| C19 | 3-CH₃O-Ph | Methyl | Methyl | Ethyl |
| C20 | 3-CH₃O-Ph | Methyl | 2-Propyt | 2-Propyl |
| C21 | 3-CH₃O-Ph | Methyl | -(CH₂)₂-O-(CH₂)₂- | |
| C22 | 3-CH₃O-Ph | Methyl | -(CH₂)₅- | |
| C23 | 3-CH₃O-Ph | Methyl | -(CH₂)₄- | |
| C24 | 3-F-Ph | Methyl | Ethyl | Ethyl |
| C25 | 3-F-Ph | Methyl | 1-Propyl | 1-Propyl |
| C26 | 3-F-Ph | Methyl | Methyl | Ethyl |
| C27 | 3-F-Ph | Methyl | 2-Propyl | 2-Propyl |
| C28 | 3-F-Ph | Methyl | -(CH₂)₂-O-(CH₂)₂- | |
| C29 | 3-F-Ph | Methyl | -(CH₂)₅- | |
| C30 | 3-F-Ph | Methyl | -(CH₂)₄- | |
| C31 | Ph | Ethyl | Ethyl | Ethyl |
| C32 | Ph | Ethyl | 1-Propyl | 1-Propyl |
| C33 | Ph | Ethyl | Methyl | Ethyl |
| C34 | Ph | Ethyl | 2-Propyl | 2-Propyl |
| C35 | Ph | Ethyl | -(CH₂)₂-O-(CH₂)₂- | |
| C36 | Ph | Ethyl | -(CH₂)₅- | |
| C37 | Ph | Ethyl | -(CH₂)₄- | |
| C38 | 3-F-Ph | Ethyl | Ethyl | Ethyl |
| C39 | 3-F-Ph | Ethyl | 1-Propyl | 1-Propyl |
| C40 | 3-F-Ph | Ethyl | Methyl | Ethyl |
| C41 | 3-F-Ph | Ethyl | 2-Propyl | 2-Propyl |
| C42 | 3-F-Ph | Ethyl | -(CH₂)₂-O-(CH₂)₂- | |
| C43 | 3-F-Ph | Ethyl | -(CH₂)₅- | |
| C44 | 3-F-Ph | Ethyl | -(CH₂)₄- | |
| C45 | Ph | Benzyl | Ethyl | Ethyl |
| C46 | 3-CH₃C(O)O- | n-Propyl | Ethyl | Ethyl |

10. The use of a compound of claim 1 in the manufacture of a medicament for use as an immunosuppressant, antiinflammatory agent, agent for the treatment of mental illness, medicament for drug and alcohol abuse, agent for treating gastritis and diarrhea, cardiovascular agent, agent for the treatment of respiratory diseases or analgesic.

11. The compounds of any one of claims 1 to 10 for use as an immunosuppressant, antiinflammatory agent, agent for the treatment of mental illness, medicament for drug and alcohol abuse, agent for treating gastritis and diarrhea, cardiovascular agent, agent for the treatment of respiratory diseases or analgesic.

12. A method to make a compound of the formula: 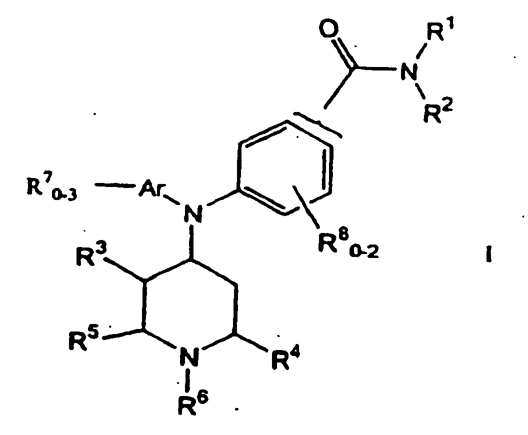 comprising the step of arylating a compound of the formula: 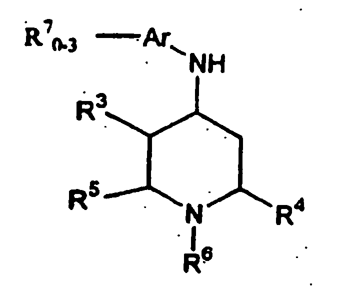 in the presence of a palladium catalyst, a phosphine ligand and a base with a compound of the forumla: 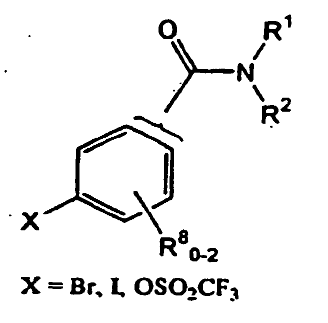 where Ar, R¹, R², R³, R⁴, R⁵, R⁶, R⁷ and R⁸ are as defined in claim 1.

## Patentansprüche

1. Verbindung, die an den Deha-Opioid-Rezeptor bindet, mit der allgemeinen Formel: 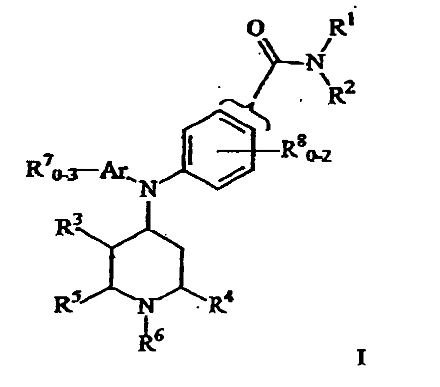 wobei
Ar Phenyl, 1-Naphthyl oder 2-Naphthyl jedes optional mit 1 bis 3 R⁷ substituiert ist;
R¹ und R² unabhängig voneinander ausgewählt sind aus der Gruppe bestehend aus Wasserstoff,
C₁₋₈Alkyl; Phenyl, optional mono-, di- oder tri-substituiert mit Halogen, C₁₋₆Alkyl, C₁₋₆Alkoxy oder Trifluormethyl; oder Benzyl, optional mono-, di- oder tri-substituiert mit Halogen, C₁₋₆Alkyl, C₁₋₆Alkoxy oder Trifluormethyl oder, alternativ, R¹ und R² mit ihrer N-Bindungsstelle zusammengenommen sind, um einen Ring zu bilden, welcher ausgewählt ist aus der Gruppe bestehend aus Pyrrolidinyl, Morpholinyl, Piperidinyl und Hexamethyleniminyl, worin jeder der Ringe optional mit 1 bis 4 Methylgruppen substituiert ist;
R³ ausgewählt ist aus Wasserstoff und C₁₋₄Alkyl;
R⁴ und R⁵ Wasserstoff sind;
R⁶ ausgewählt ist aus der Gruppe bestehend aus Wasserstoff; C₁₋₈Alkyl; C₃₋₆Cycloalkyl-C₁₋₃alkyl; C₃₋₆Alkenyl; C₁₋₆Alkoxy-C₁₋₃alkyl und Phenyl-C₁₋₄alkyl, worin das Phenyl optional mit R⁷ mono-, di- oder tri-substituiert ist;
R⁷ unabhängig ausgewählt ist aus der Gruppe bestehend aus Hydroxyl, Halogen, C₁₋₃Alkyl, C₁₋₃Alkoxy, C₁₋₃Acyl, C₁₋₃Acyloxy, Cyano, Amino, C₁₋₃Acylamino, C₁₋₃Alkylamino, di(C₁₋₃Alkyl)amino, C₁₋₃Alkylthio, C₁₋₃Alkylsulfonyl, Trifluormethyl und Trifluormethoxy, und zwei R⁷ eine einzige Gruppe bilden können, die ausgewählt ist aus der Gruppe bestehend aus -(CH₂)₃₋₅- und -O(CH₂)₁₋₃O- gebunden an benachbarte Kohlenstoffatome von Ar; und
R⁸ unabhängig ausgewählt ist aus der Gruppe bestehend aus Halogen, C₁₋₆Alkyl, C₁₋₆Alkoxy und Trifluormethyl;
wobei die Verbindungen der Formeln: 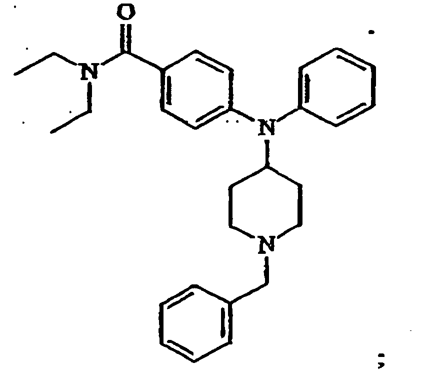 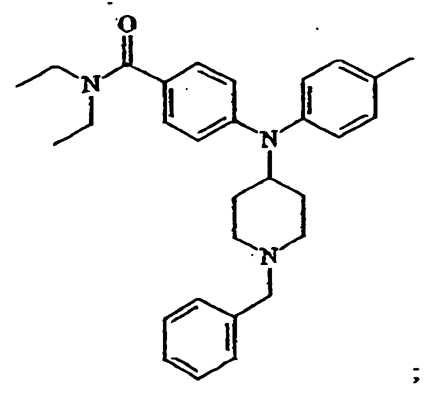 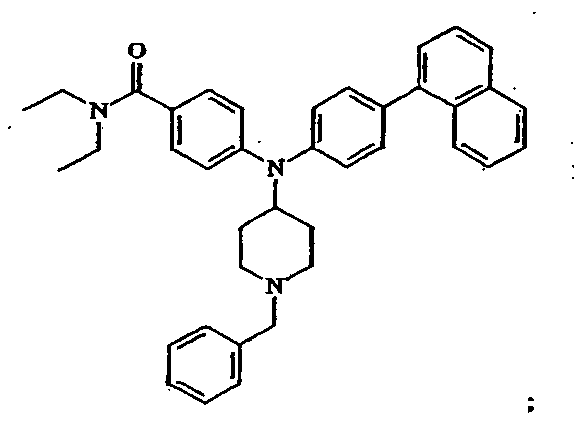 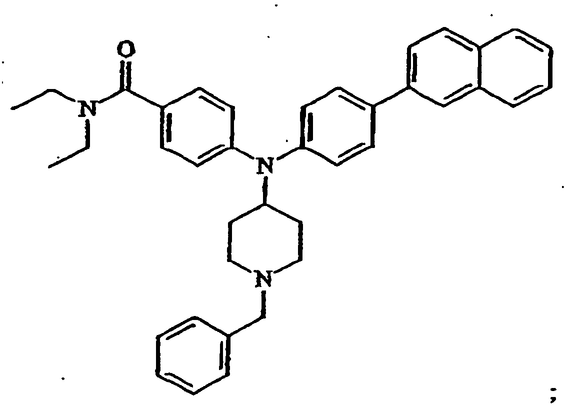 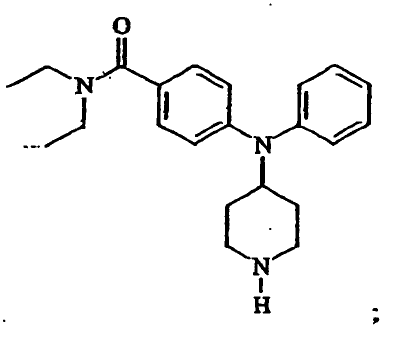 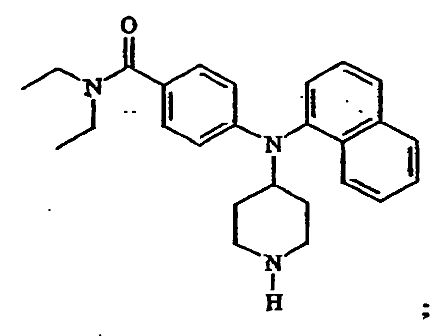 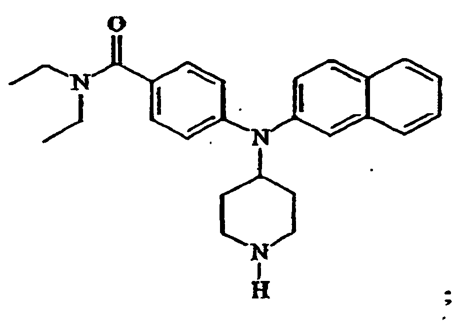 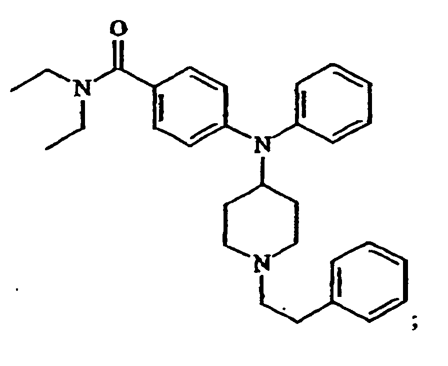 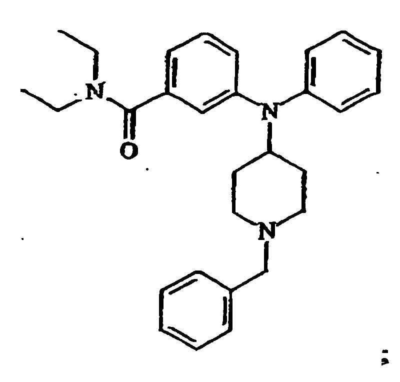 und 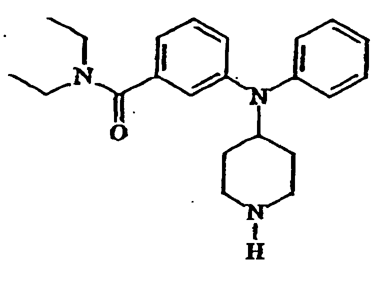 ausgenommen sind.

2. Verbindung nach Anspruch 1, worin Ar Phenyl ist

3. Verbindung nach Anspruch 1, worin R¹ und R² unabhängig ausgewählt sind aus der Gruppe bestehend aus Wasserstoff, Methyl, Ethyl, n-Propyl, i-Propyl, n-Butyl, t-Butyl, i-Butyl, Phenyl, p-Chlorphenyl, p-Fluvrphenyl, p-Methylphenyl, p-Trifluormethylphenyl, Benzyl, p-Chlorbenzyl, p-Fluorbenzyl, p-Methylbenzyl und p-Trifluormethylbenzyl oder alternativ R¹ und R² mit ihrer N-Bindungsstelle zusammengenommen sind, um einen Ring zu bilden, welcher ausgewählt ist aus der Gruppe bestehend aus Pyrrolidinyl und Piperidinyl.

4. Verbindung nach Anspruch 1, worin R³ ausgewählt ist aus Wasserstoff, Methyl, Ethyl, n-Propyl, i-Propyl und t-Butyl.

5. Verbindung nach Anspruch 1, worin R⁶ ausgewählt ist aus der Gruppe bestehend aus Wasserstoff, Methyl, Ethyl, n-Propyl, i-Propyl, n-Butyl, i-Butyl, t-Butyl, Cyclopropylmethyl, Ethenyl, Allyl, Methoxymethyl, Benzyl, p-Chlorbenzyl, p-Fluorbenzyl, p-Methylbenzyl, p-Trifluormethylbenzyl, p-Aminobenzyl und Phenyl-CH₂CH₂-.

6. Verbindung nach Anspruch 1, worin R⁷ unabhängig ausgewählt ist aus der Gruppe bestehend aus Hydroxyl, Chlor, Brom, Fluor, Methyl, Ethyl, n-Propyl, i-Propyl, n-Butyl, i-Butyl, t-Butyl, Methoxy, Ethoxy, Formyl, Acyl, Acetoxy, Cyano, Amino, Methylamido, Methylamino, N,N-Dimethylamino, Methylthio, Methylsulfonyl, Trifluormethoxy und Trifluormethyl und bevorzugte Gruppen, in denen zwei R⁷ zusanonen eine einzelne Gruppe bilden, ausgewählt sind aus der Gruppe bestehend aus Propylen, Butylen und -OCH₂O-.

7. Verbindung nach Anspruch 1, wobei R⁸ unabhängig ausgewählt ist aus der Gruppe bestehend aus Chlor, Brom, Fluor, Methyl, Ethyl, n-Propyl, i-Propyl, t-Butyl, Methoxy, Ethoxy und Trifluormethyl.

8. Verbindung nach Anspruch 1, die die allgemeine Struktur: 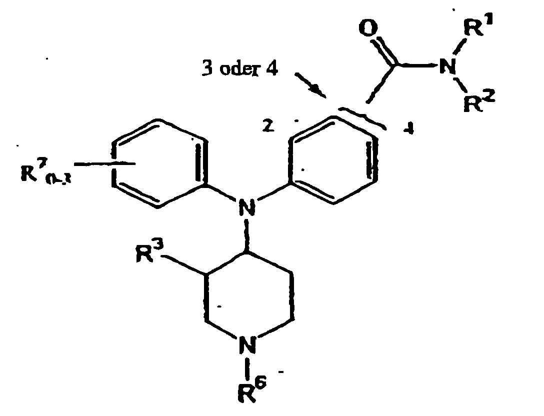 aufweist, worin R¹, R², R³, R⁶ und R⁷ abhängig voneinander ausgewählt sind aus der Gruppe bestehend aus:

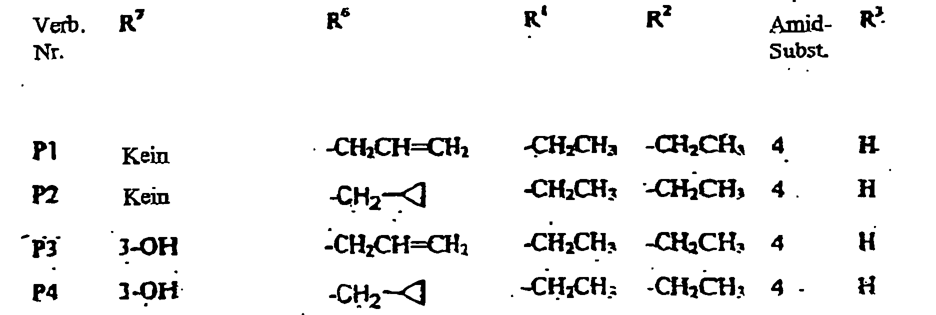

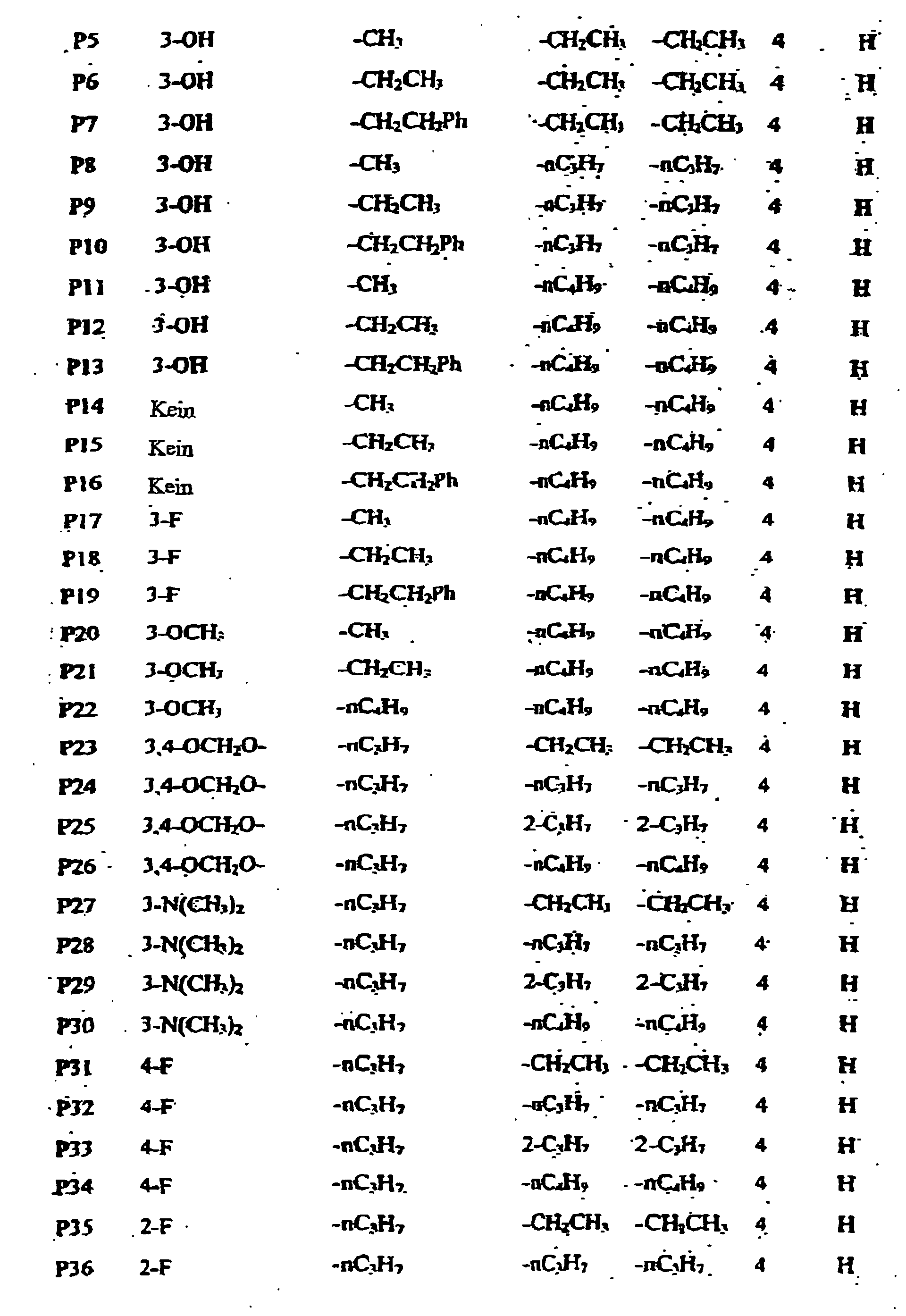

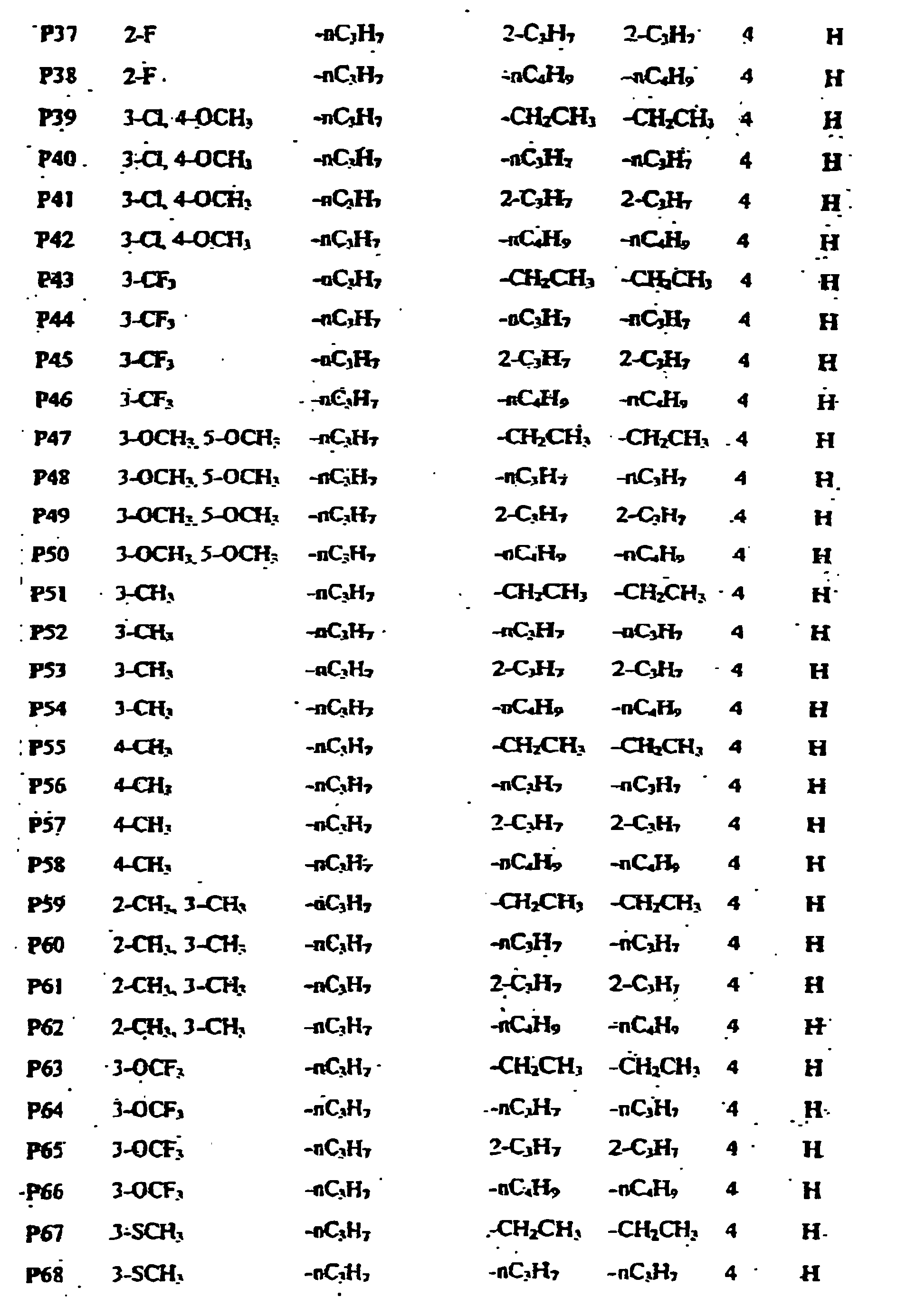

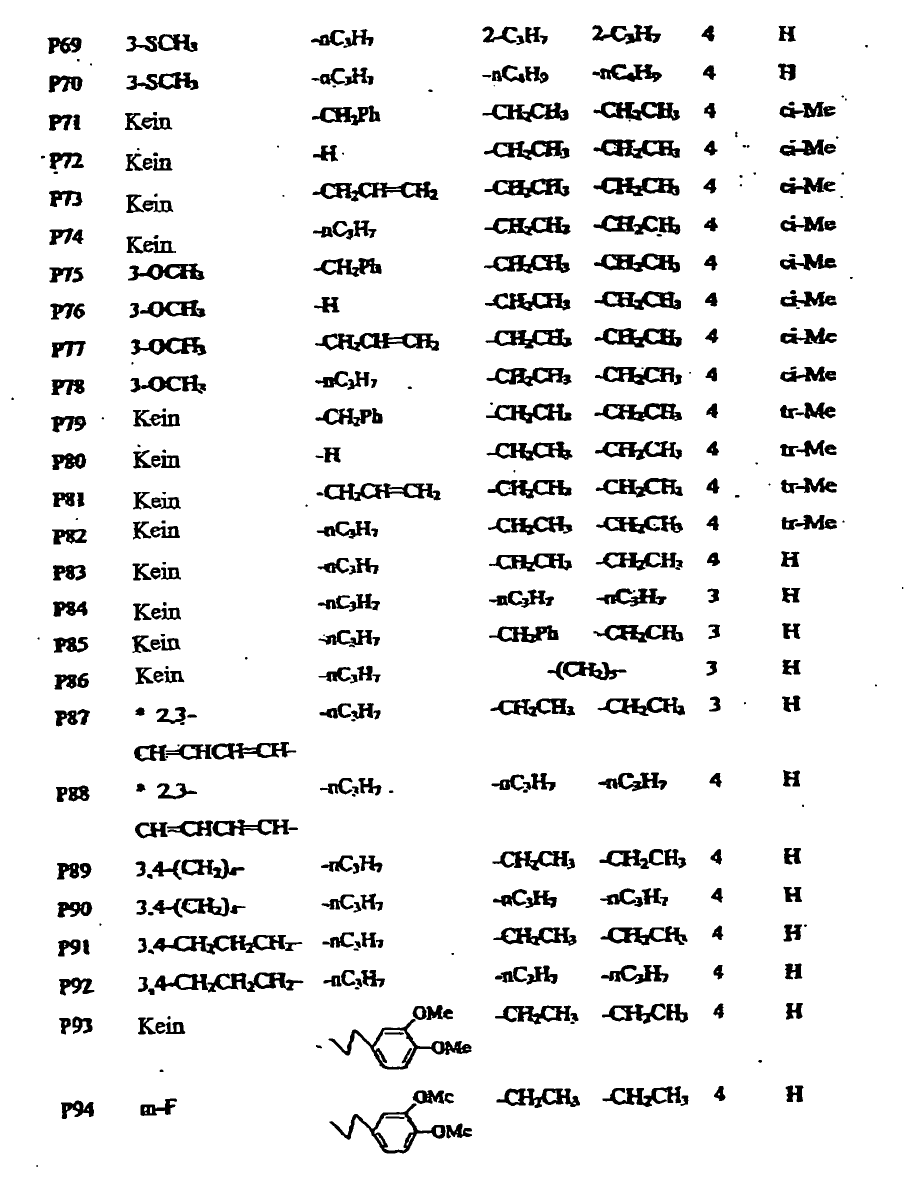

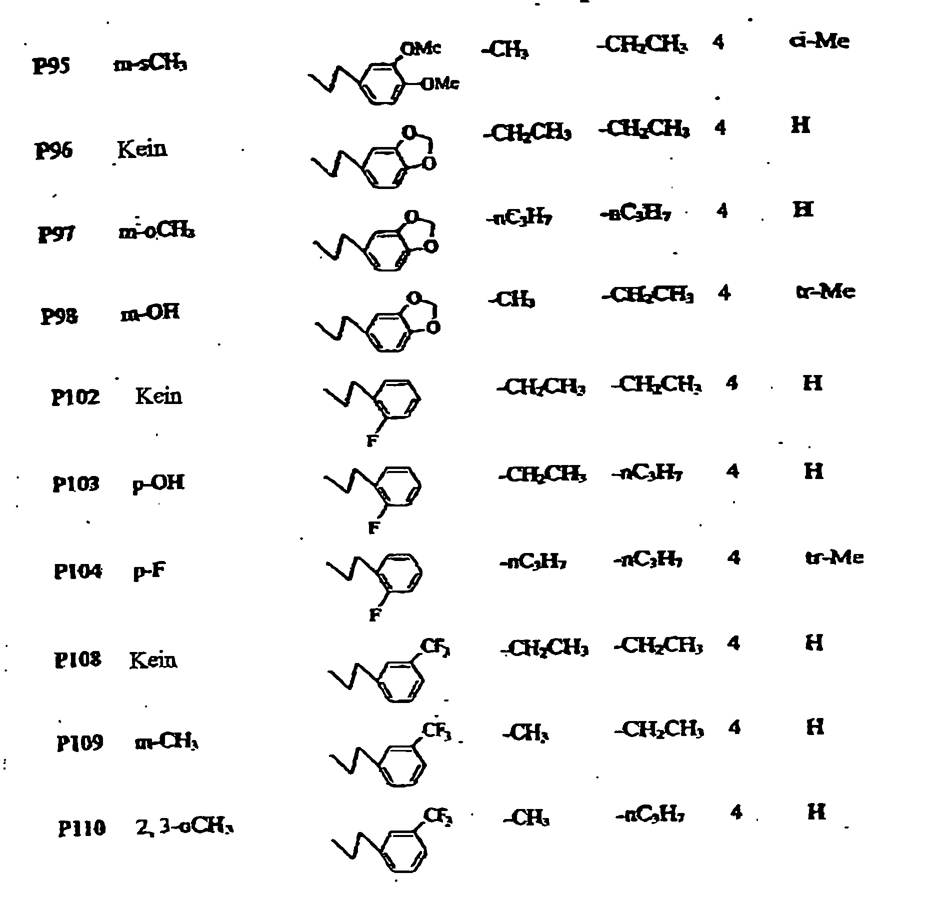

9. Verbindung nach Anspruch 1, die die allgemeine Struktur: 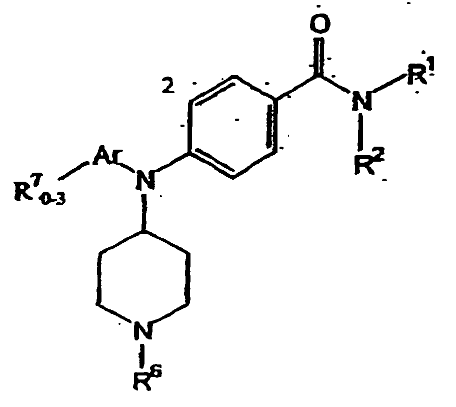 aufweist, worin R¹, R², R⁶ und R⁷-Ar abhängig voneinander ausgewählt sind aus den Gruppen bestehend aus:
| Verb. Nr. | R⁷-Ar | R⁶ | R¹ | R² |
|---|---|---|---|---|
| C1 | 3-CH₃O-Ph | 1-Propyl | Ethyl | Ethyl |
| C2 | 3-HO-Ph | 1-Propyl | Ethyl | Ethyl |
| C3 | Ph | 1-Propyl | Ethyl | Ethyl |
| C4 | Ph | 1-Propyl | Methyl | Ph |
| C5 | 3-Cl-Ph | 1-Propyl | Ethyl | Ethyl |
| C6 | 3-Cl-Ph | 1-Propyl | -(CH₂)₄- | |
| C7 | 2-CH₂O-Ph | 1-Propyl | Ethyl | Ethyl |
| C8 | 1-Naphthyl | 1-Propyl | Ethyl | Ethyl |
| C9 | 1-Naphthyl | 1-Propyl | -(CH₂)₄- | |
| C10 | Ph | Methyl | Ethyl | Ethyl |
| C11 | Ph | Methyl | 1-Propyl | 1-Propyl |
| C12 | Ph | Methyl | Methyl | Ethyl |
| C13 | Ph | Methyl | 2-Propyl | 2-Propyl |
| C14 | Ph | Methyl | -(CH₂)₂-O-(CH₂)₂- | |
| C15 | Ph | Methyl | -(CH₂)₃- | |
| C16 | Ph | Methyl | -(CH₂)₄- | |
| C17 | 3-CH₃O-Ph | Methyl | Ethyl | Ethyl |
| C18 | 3-CH₃O-Ph | Methyl | 1-Propyl | 1-Propyl |
| C19 | 3-CH₃O-Ph | Methyl | Methyl | Ethyl |
| C20 | 3-CH₃O-Ph | Methyl | 2-Propyl | 2-Propyl |
| C21 | 3-CH₃O-Ph | Methyl | -(CH₂)₂-O-(CH₂)₂- | |
| C22 | 3-CH₃O-Ph | Methyl | -(CH₂)₅- | |
| C23 | 3-CH₃O-Ph | Methyl | -(CH₂)₄- | |
| C24 | 3-F-Ph | Methyl | Ethyl | Ethyl |
| C25 | 3-F-Ph | Methyl | 1-Propyl | 1-Propyl |
| C26 | 3-F-Ph | Methyl | Methyl | Ethyl |
| C27 | 3-F-Ph | Methyl | 2-Propyl | 2-Propyl |
| C28 | 3-F-Ph | Methyl | -(CH₂)₂-O-(CH₂)₂- | |
| C29 | 3-F-Ph | Methyl | -(CH₂)₃- | |
| C30 | 3-F-Ph | Methyl | -(CH₂)₄- | |
| C31 | Ph | Ethyl | Ethyl | Ethyl |
| C32 | Ph | Ethyl | 1-Propyl | 1-Propyl |
| C33 | Ph | Ethyl | Methyl | Ethyl |
| C34 | Ph | Ethyl | 2-Propyl | 2-Propyl |
| C35 | Ph | Ethyl | -(CH₂)₂-O-(CH₂)₂- | |
| C36 | Ph | Ethyl | -(CH₂)₃- | |
| C37 | Ph | Ethyl | -(CH₂)₃- | |
| C38 | 3-F-Ph | Ethyl | Ethyl | Ethyl |
| C39 | 3-F-Ph | Ethyl | 1-Propyl | 1-Propyl |
| C40 | 3-F-Ph | Ethyl | Methyl | Ethyl |
| C41 | 3-F-Ph | Ethyl | 2-Propyl | 2-Propyl |
| C42 | 3-F-Ph | Ethyl | -(CH₂)₂-O-(CH₂)₂- | |
| C43 | 3-F-Ph | Ethyl | -(CH₂)₅- | |
| C44 | 3-F-Ph | Ethyl | -(CH₂)₃- | |
| C45 | Ph | Benzyl | Ethyl | Ethyl |
| C46 | 3-CH₃C(O)O- | n-Propyl | Ethyl | Ethyl |

10. Verwendung einer Verbindung nach Anspruch 1 zur Herstellung eines Medikaments zur Verwendung als ein Immunsuppressivum, antiinflammatorisches Mittel, Mittel für die Behandlung von mentaler Erkrankung, Medikament für Drogen- und Alkoholmißbrauch, Mittel für die Behandlung von Gastritis und Diarrhöe, kardiovaskuläres Mittel, Mittel für die Behandlung von respiratorischen Krankheiten oder Analgetikum.

11. Verbindungen nach einem der Ansprüche 1 bis 10 zur Verwendung als ein Immunosuppressivum, antünflammotorisches Mittel, Mittel für die Behandlung von mentaler Erkrankung, Medikament für Drogen- und Alkoholmißbrauch, Mittel für die Behandlung von Gastritis und Diarrhöe, kardiovaskuläres Mittel, Mittel für die Behandlung von respiratorischen Krankheiten oder Analgetikum.

12. Verfahren zur Herstellung einer Verbindung der Formel: 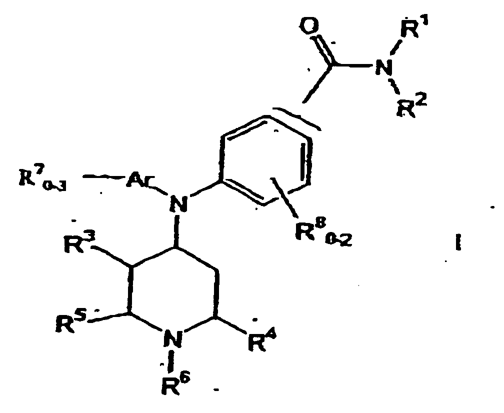 umfassend den Schritt der Arylienmg einer Verbindung der Formel: 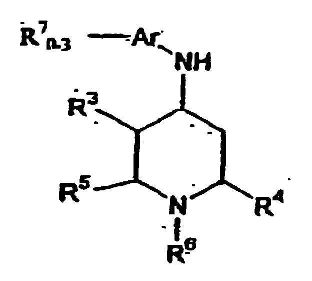 in Anwesenheit eines Palladium-Katalysators, eines Phosphin-Liganden und einer Base mit einer Verbindung der Formel: 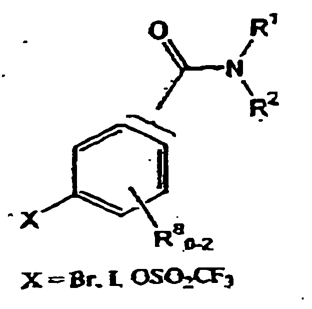 worin Ar, R¹, R², R³, R⁴, R⁵, R⁶, R⁷ und R⁸ wie in Anspruch 1 definiert sind.

## Revendications

1. Composé qui se lie au récepteur opioïde-delta de la formule générale: 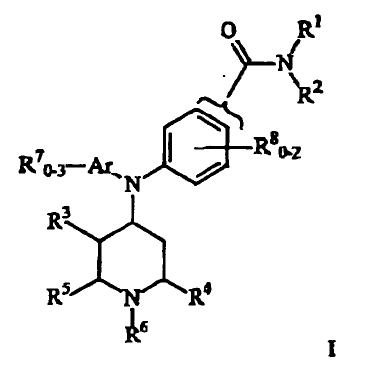 où
Ar est phényle, 1-naphtyle ou 2-naphtyle, chacun facultativement substitué par 1 à 3 R⁷;
R¹ et R² sont indépendamment sélectionnés dans le groupe consistant en hydrogène, alkyle C₁₋₈; phényle, facultativement mono-, di-, ou tri-substitué par halo, alkyleC₁₋₆, alcoxyC₁₋₆ ou trifluorométhyle; ou bien benzyle, facultativement mono-, di-, ou tri-substitué par halo, alkyle C₁₋₆, alcoxy C₁₋₆ ou trifluorométhyle,, ou alternativement, R¹ et R² sont pris ensemble avec leur N d'attachement pour former un cycle qui est sélectionné dans le groupe consistant en pyrrolidinyle, morpholinyle, pipéridinyle et hexaméthylèneiminyle, chacun desdits cycles étant facultativement substitué par 1 à 4 groupes méthyles;
R³ est sélectionné parmi hydrogène et alkyleC₁₋₄;
R⁴ et R⁵ sont hydrogène;
R⁶ est sélectionné dans le groupe consistant en hydrogène; alkyleC₁₋₈, cycloalkylC₃₋₆ alkyle C₁₋₃; alcényle C₃₋₆; alcoxyC₁₋₆alkyleC₁₋₃ et phénylalkyleC₁₋₄, où le phényle est facultativement mono-, di-, ou tri-substitué par R⁷;
R⁷ est indépendamment sélectionné dans le groupe consistant en hydroxy, halo, alkyleC₁₋₃, alcoxyC₁₋₃, acyleC₁₋₃, acyloxyC₁₋₃, cyano, amino, acylaminoC₁₋₃, alkylaminoC₁₋₃, di(alkylC₁₋₃)amino, alkylthioC₁₋₃, alkylsulfonyleC₁₋₃, trifluorométhyle et trifluorométhoxy, et deux R⁷ peuvent ensemble former une seule fraction sélectionnée dans le groupe consistant en -(CH₂)₃₋₅- et -O(CH₂)₁₋₃O- attaché à des atomes de carbone adjacents de Ar; et
R⁸ est indépendamment sélectionné dans le groupe consistant en halo, alkyle C₁₋₆, alcoxy C₁₋₆ et trifluorométhyle;
où les composés des formules: 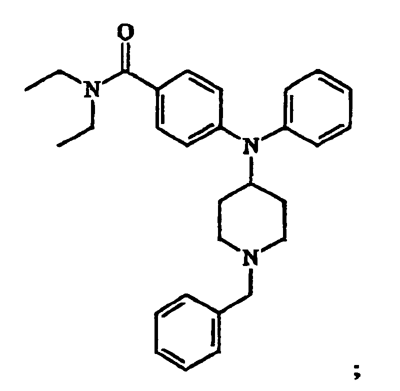 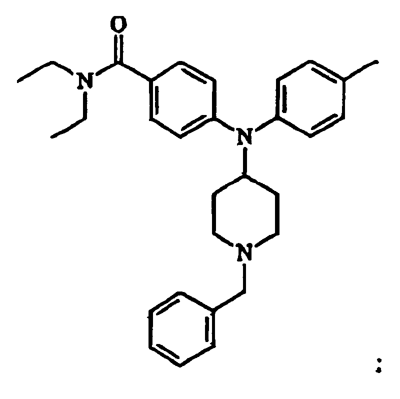 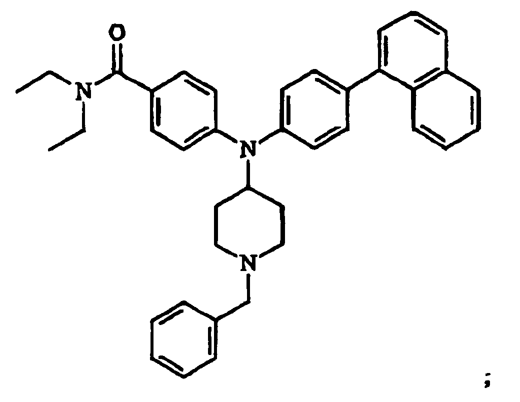 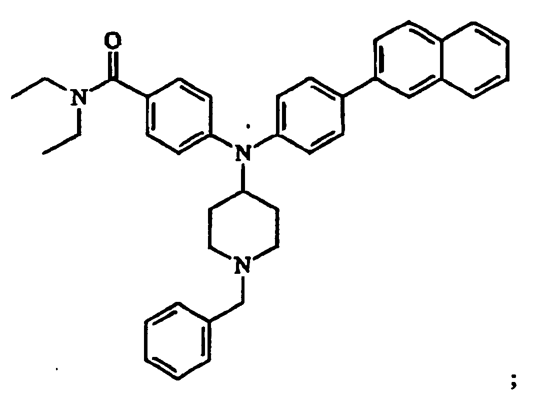 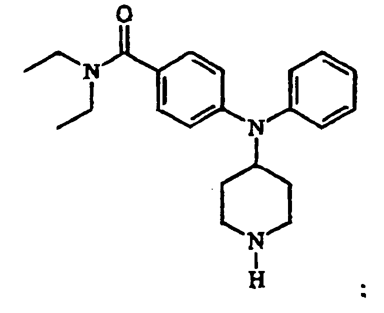 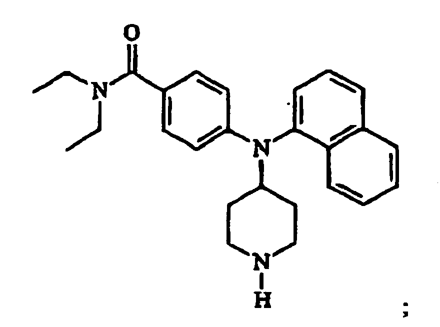 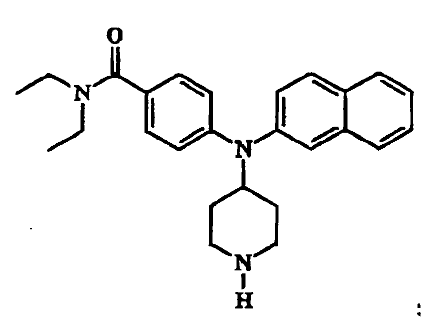 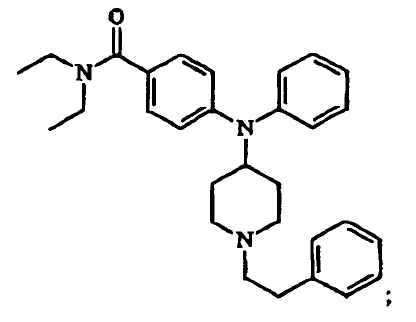 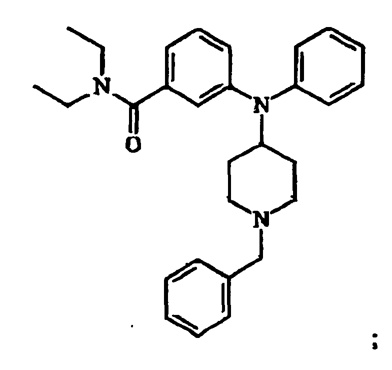 et 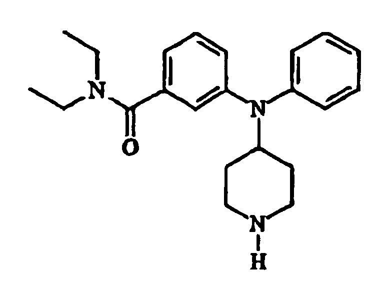 ne sont pas revendiqués.

2. Composé de la revendication 1 où Ar est phényle.

3. Composé de la revendication 1 où R¹ et R² sont indépendamment sélectionnés dans le groupe consistant en hydrogène, méthyle, éthyle, n-propyle, i-propyle, n-butyle, t-butyle, i-butyle, phényle, p-chlorophényle, p-fluorophényle, p-anéthylphényle, p-trifluorométhylphényle, benzyle, p-chlorobenzyle, p-fluorobenzyle, p-méthylbenzyle et p-trifluorométhylbenzyle, ou, alternativement, R¹ et R² sont pris ensemble avec leurs N d'attachement pour former un cycle qui est sélectionné dans le groupe consistant en pyrrolidinyle et pipéridinyle.

4. Composé de la revendication 1 où R³ est sélectionné parmi hydrogène, méthyle, éthyle, n-propyle, i-propyle et t-butyle.

5. Composé de la revendication 1 où les R⁶ sont sélectionnés dans le groupe consistant en hydrogène, méthyle, éthyle, n-propyle, i-propyle, n-butyle, i-butyle, t-butyle, cyclopropylméthyle, éthényle, allyle, méthoxyméthyle, benzyle, p-chlorobenzyle, p-fluorobenzyle, p-méthylbenzyle, p-trifluorométhylbenzyle, p-aminonbenzyle et phényl CH₂CH₂-.

6. Composé de la revendication 1 où les R⁷ sont indépendamment sélectionnés dans le groupe consistant en hydroxy, chloro, bromo, fluoro, méthyle, éthyle, n-propyle, i-propyle, n-butyle, i-butyle, t-butyle, méthoxy, éthoxy, formyle, acyle, acétoxy, cyano, amino, méthylamido, méthylamino, N,N-diméthylamino, mêthylthio, méthylsulfonyle, trifluorométhoxy et trifluorométhyle, et les fractions préférées où deux R⁷ forment ensemble une seule fraction sont sélectionnés dans le groupe consistant en propylène, butylène, et -OCH₂O-.

7. Composé de la revendication 1 où les R⁸ sont indépendamment sélectionnés dans le groupe consistant en chloro, bromo, fluoro, méthyle, éthyle, n-propyle, i-propyle, t-butyle, méthoxy, éthoxy et trifluorométhyle.

8. Composé de la revendication 1 ayant la structure générale: 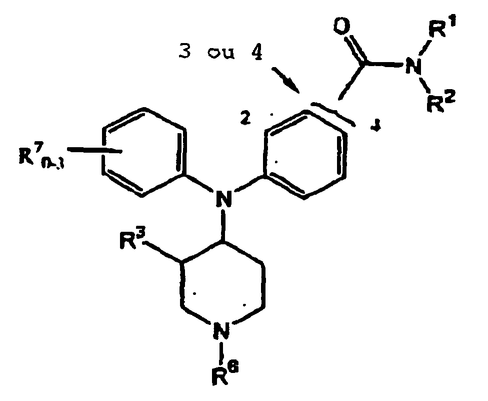 où R¹,R²,R³,R⁶ et R⁷ sont dépendamment sélectionnés dans les groupes consistant en:

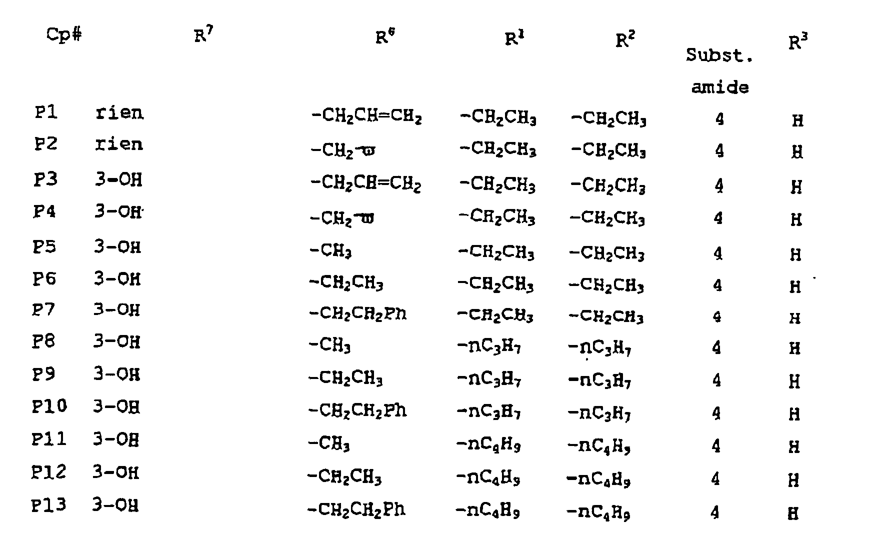

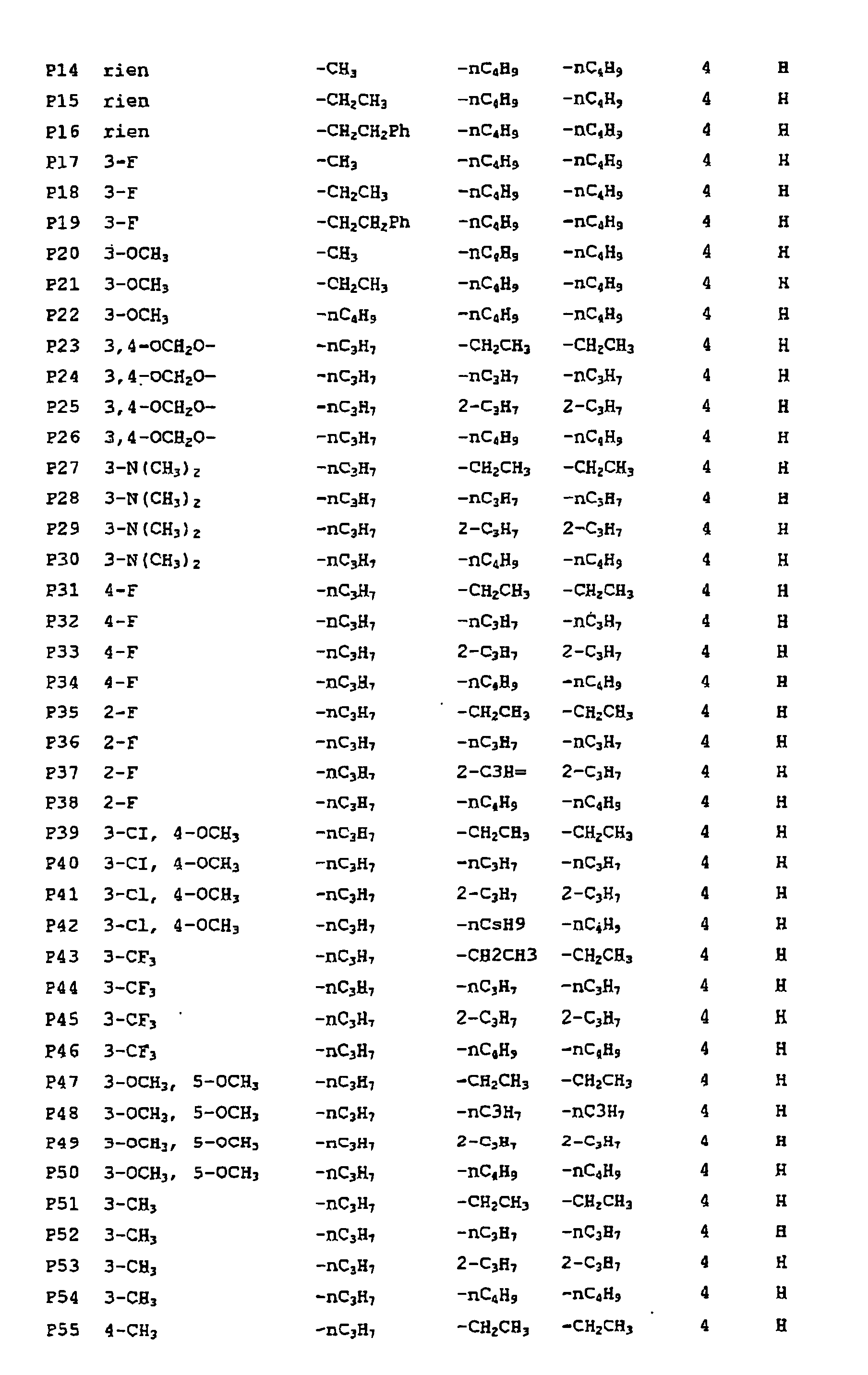

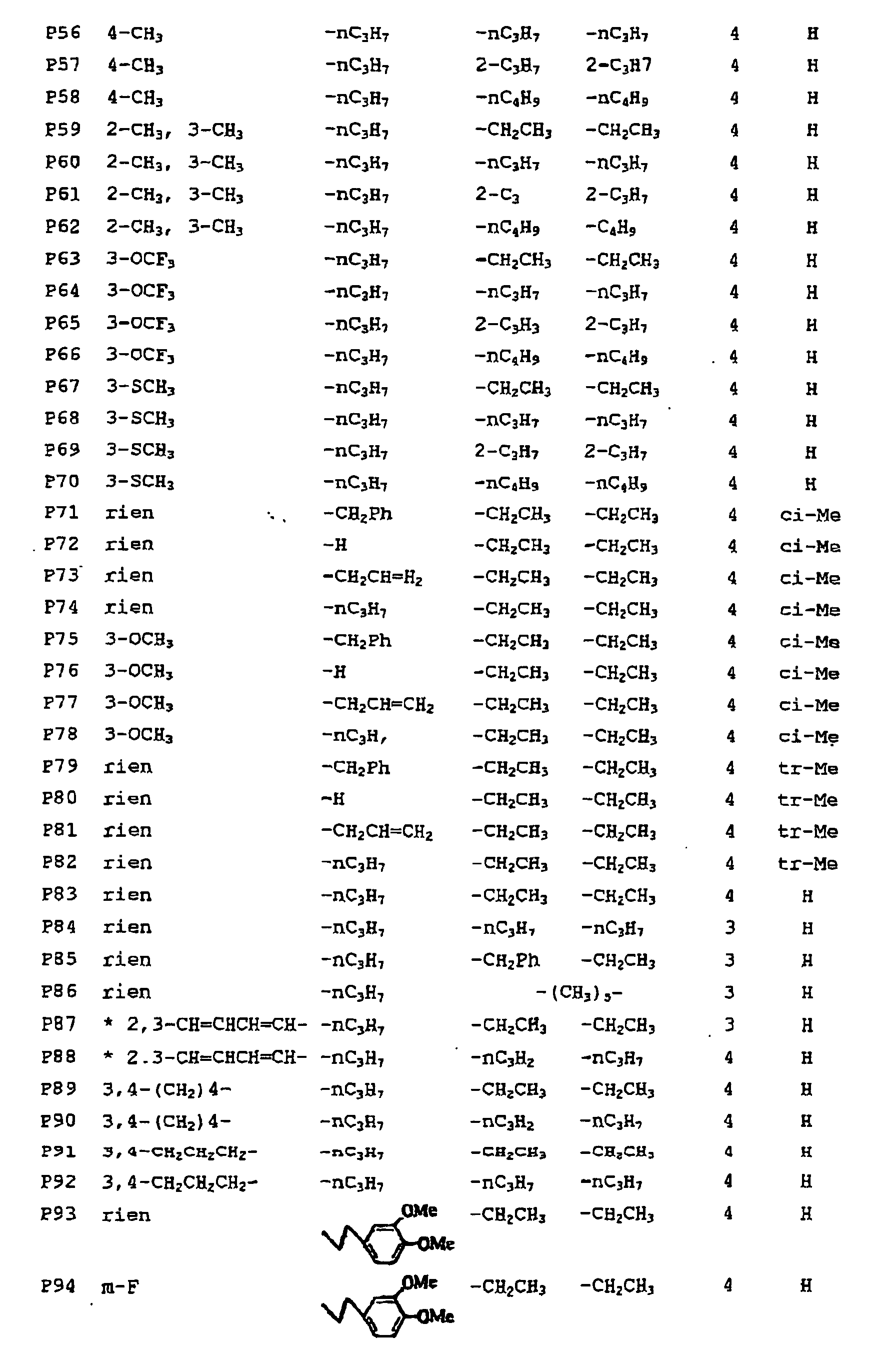

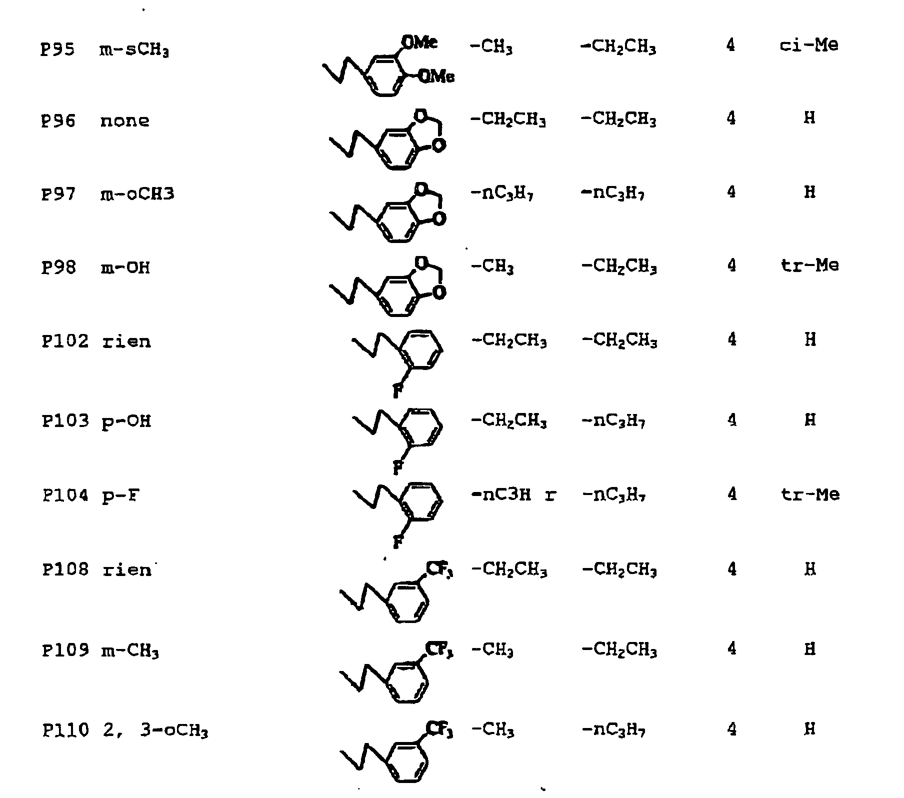

9. Composé de la revendication 1 ayant la structure générale: 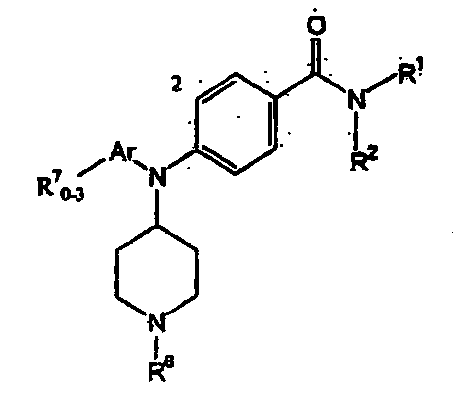 où R¹,R²,R⁶ et R⁷-Ar sont dépendamment sélectionnés dans les groupes consistant en:
| Cp# | R⁷-Ar | R⁶ | R¹ | R² |
|---|---|---|---|---|
| C1 | 3-CH₃O-Ph | 1-Propyle | Ethyle | Ethyle |
| C2 | 3-HO-Ph | 1-Propyle | Ethyle | Ethyle |
| C3 | Ph | 1-Propyle | Ethyle | Ethyle |
| C4 | Ph | 1-Propyle | Methyle | Ph |
| C5 | 3-CI-Ph | 1-Propyle | Ethyle | Ethyle |
| C6 | 3-CI-Ph | 1-Propyle | -(CH₂)₄- | |
| C7 | 2-CH₃O-Ph | 1-Propyle | Ethyle | Ethyle |
| C8 | 1-Naphtyle | 1-Propyle | Ethyle | Ethyle |
| C9 | 1-Naphtyle | 1-Propyle | -(CH₂)₄- | |
| C10 | Ph | Methyle | Ethyle | Ethyle |
| C11 | Ph | Methyle | I-Propyle | 1I-Propyle |
| C12 | Ph | Methyle | Methyle | Ethyle |
| C13 | Ph | Methyle | 2-Propyle | 2-Propyle |
| C14 | Ph | Methyle | -(CH₂)2-O-(CH₂)- | |
| C15 | Ph | Methyle | -(CH₂)₅- | |
| C16 | Ph | Methyle | -(CH₂)₄- | |
| C17 | 3-CH₃O-Ph | Methyle | Ethyle | Ethyle |
| C18 | 3-CH₃O-Ph | Methyle | I-Propyle | 1-Propyle |
| C19 | 3-CH₃O-Ph | Methyle | Methyle | Ethyle |
| C20 | 3-CH₃O-Ph | Methyle | 2-Propyle | 2-Propyle |
| C21 | 3-CH₃O-Ph | Methyle | -(CH₂)₂-O-(CH₂)₂- | |
| C22 | 3-CH₃O-Ph | Methyle | -(CHj)₅- | |
| C23 | 3-CH₃O-Ph | Methyle | -(CH2)₄- | |
| C24 | 3-F-Ph | Methyle | Ethyle | Ethyle |
| C25 | 3-F-Ph | Methyle | 1-Propyle | 1-Propyle |
| C26 | 3-F-Ph | Methyle | Methyle | Ethyle |
| C27 | 3-F-Ph | Methyle | 2-Propyle | 2-Propyle |
| C28 | 3-F-Ph | Methyle | -(CH₂)₂-O- (CH₂)₂- | |
| C29 | 3-F-Ph | Methyle | -(CH₂)₅- | |
| C30 | 3-F-Ph | Methyle | -(CH₂)₄- | |
| C31 | Ph | Ethyle | Ethyle | Ethyle |
| C32 | Ph | Ethyle | 1-Propyle | 1-Propyle |
| C33 | Ph | Ethyle | Methyle | Ethyle |
| C34 | Ph | Ethyle | 2-Propyle | 2-Propyle |
| C35 | Ph | Ethyle | -(CH₂)₂-O-(CH₂)₂- | |
| C36 | Ph | Ethyle | -(CH₂)₅- | |
| C37 | Ph | Ethyle | -(CH₂)₄- | |
| C38 | 3-F-Ph | Ethyle | Ethyle | Ethyle |
| C39 | 3-F-Ph | Ethyle | 1-Propyle | 1-Propyle |
| C40 | 3-F-Ph | Ethyle | Methyle | Ethyle |
| C41 | 3-F-Ph | Ethyl | 2-Propyle | 2-Propyle |
| C42 | 3-F-Ph | Ethyle | -(CH₂)₂-O- (CH₂)₂- | |
| C43 | 3-F-Ph | Ethyle | -(CH₂)₅- | |
| C44 | 3-F-Ph | Ethyle | -(CH₂)₄- | |
| C45 | Ph | Benzyle | Ethyle | Ethyle |
| C46 | 3-CH₃C(O)O- | n-Propyle | Ethyle | Ethyle |

10. Utilisation d'un composé de la revendication 1 dans la fabrication d'un médicament à utiliser comme immunosuppresseur, agent antiinflammatoire, agent pour le traitement des maladies mentales, médicament pour l'abus de drogues et d'alcools, agents pour le traitement de la gastrite et de la diarrhée, agent cardiovasculaire, agents pour le traitement des maladies respiratoires ou analgésique.

11. Composé de l'une quelconque des revendications 1 à 10 à utiliser comme immunosuppresseur, agent antiinflammatoire, agent pour le traitement des maladies mentales, médicament pour l'abus des drogues et des alcools, agents pour le traitement de la gastrite et de la diarrhée, agent cardiovasculaire, agent pour le traitement des maladies respiratoires ou analgésique.

12. Méthode de production d'un composé de la formule: 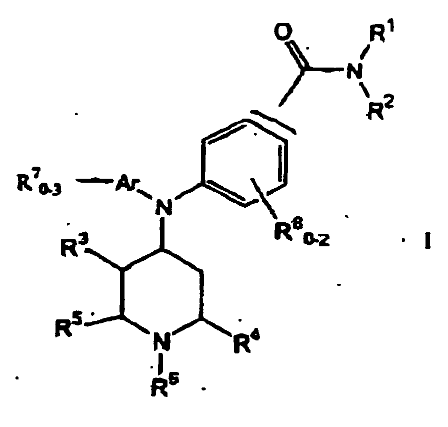 comprenant l'étape d'aryler un composé de la formule: 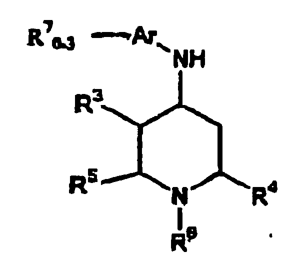 en présence d'un catalyseur de palladium, un ligand de phosphine et une base avec un composé de la formule: 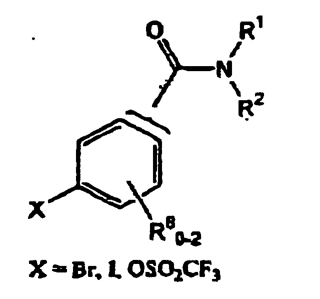 où Ar, R¹, R², R³, R⁴, R⁵, R⁶, R⁷ et R⁸ sont tels que définis à la revendication 1.
